# EUROPEAN PATENT APPLICATION

(11) **EP 2 508 535 A1**
(43) Date of publication of application: **10.10.2012**
(21) Application number: 12174478.3
(22) Date of filing: 12.09.2007
(51) Int. Cl.: C07K 14/47, C12N 15/79, C12N 15/63, C12N 15/861, A61K 48/00, C12Q 1/68

(54) **Nucleic acids for expressing a polynucleotide of interest in mammalian cancer cells**

(30) Priority: 12.09.2006 EP 06300942
(62) Divisional of application: 07803443.6
(71) Applicant: INSERM (Institut National de la Santé et de la Recherche Médicale), 75013 Paris (FR)
(72) Inventor: Faivre, Jamila, 75016 Paris (FR); Herve, Julie, 49000 Angers (FR); Clerc, Jérôme, 75005 Paris (FR); Brechot, Christian, 75015 Paris (FR)
(74) Representative: Coralis Harle

(57) **Abstract**

The invention relates to regulatory polynucleotides derived (i) from rat and human AFP promoter sequences and (ii) from rat and human HIP/PAPI promoter sequences, which regulatory polynucleotides are useful for expressing nucleic acids of interest specifically in mammalian cancer cells, including mammalian cancer liver cells.

## Description

### FIELD OF THE INVENTION

The present invention relates to the field of the therapeutical treatment of cancers.

### BACKGROUND OF THE INVENTION

Methods for treating cancer have improved greatly over the years. Improved diagnostic methods combined with better surgical techniques have allowed surgeons to remove a tumour more safely by removing a minimal amount of normal tissue. The recovery time of patients has been decreased, and psychological impact due to cosmetic trauma reduced. However, while surgery is useful for treating patients whose tumours are localised, or have only minimally spread, for example, to local lymph nodes, it has but a limited usefulness for patients with metastatic diseases, or with systemic cancers such as leukaemia or lymphoma.

In the United States, approximately 15,300 people (with a 2:1 male to female ratio) are diagnosed with primary liver cancer every year. Primary liver cancer is relatively rare in North America and Europe. It has an estimated of incidence of 3 per 100,000 in the U.S. Nevertheless, it poses a serious health problem worldwide. Its incidence in epidemic regions such as Africa and Asia varies from 30 per 100,000 to more than 100 per 100,000 (American Cancer Society, Cancer Facts and Figures, 1994, Atlanta, Ga.: American Cancer Society Inc.). In the U.S, although it ranks low in the scale of incidence of cancers, primary liver cancer is a serious concern since it carries a grave prognosis with a fatality-to-case ratio of 0.8 (about 13,800 out of the 15,300 US patients affected by primary liver cancer yearly die).

Over past decades the understanding of the molecular mechanisms of cancer has grown very rapidly. The challenge is to translate the growing understanding of the molecular basis of cancer into improvements in the treatment of cancer. It is now generally admitted that tumour development in humans is a multi-step process. These steps correspond to specific genetic alterations, which drive the progressive transformation of normal human cells to highly malignant derivatives that are characterised by increased cell proliferation, decreased cell death, genomic instability and sustained angiogenesis (Hanahan D., Weinberg R. A. 2000; Cell 100: 57-70). These new insights have suggested new therapeutic approaches, which specifically target the molecular interactions and biochemical pathways that are altered in tumour cells.

Drug delivery systems usually involve devices or carriers so designed as to slowly release drugs into wide anatomical compartments, such as the gastrointestinal tract, the genito-urinary tract or the blood circulation. For example, liposomes are designed to release drugs into the blood circulation. The problem with such systems is that they are not designed to deliver drugs specifically to tumour cells (Schally & Nagy, Europ J Endoctinol 1999; 141:1). Drugs are delivered indiscriminately to normal and tumour cells. Therefore, much larger doses of drugs must be used. Since anti-cancer drugs are toxic, the use of larger drug doses causes serious clinical problems. Cancer patients often suffer from the side effects of their treatment nearly as much as from the effects of their cancer. Nevertheless, the predominant trend in cancer treatment is still to use more drugs.

The problem of drug toxicity might be solved by developing drug delivery systems capable of delivering anti-cancer drugs only to cancer cells, not to normal cells. An approach to this problem has been to use drug-laden antibodies that recognise, and react with, antigens specific of cancer cells. There are many such antigens, some of which have been used in cancer treatment paradigms.

For example, transferrin receptor antigens are present on the surface of cancer cells, not on the surface of most normal cells (Whitney et al., Cancer 1995; 76: 20). Anticancer drugs have been coupled to anti-transferrin receptor antibodies, and thus delivered to patients presenting with brain cancer (Laske et al., Neurosurg 1997;41:1039). Similar strategies have been applied with isotope-labelled antibodies in patients with lymphomes (Vose et al., Leukemia & Lymphoma 2000; 38: 91). This antibody-mediated approach to drug delivery is burdened with several serious problems. Antibodies, which are produced in genetically modified animals, can cause undesirable reactions in patients (Muraszko et al., Cancer Res 1983; 53: 3752).

A safer approach to the problem of drug delivery in anticancer therapy is to design drug carriers using the normal ligands for the receptors on cancer cells. A ligand is a molecule that fits uniquely into a specific receptor, analogous to a key that fits uniquely into a specific lock. The requirement for this approach is that the receptor be present on the surface of the sole cancer cells. Such is the case for transferrin receptors, which bind the protein ligand involved in iron transport in blood. This protein ligand has been modified to carry anticancer drugs, and the efficacy of this ligand-receptor drug delivery system in the treatment of acute leukaemia has been demonstrated (Faulk et al., Mol Biotherapy 1990; 2: 57).

Drug-carrying ligands are also capable of carrying several different types of anticancer drugs (Berczi et al., Arch Biochem Biophy 1993; 300: 356, and Beyer et al., J Med Chem 1998; 41: 2701) through various types of chemical bonds (Kratz et al., J Pharm Sci 1998; 87: 338). However, this possibility is limited to protein ligands involved in the transport of heavy metals, such as iron.

Strategies for anticancer therapy relying on tumour cell-selective transgene expression are also under study (Binley K. et al., (1999) Gene Therapy 6: 1721-1727; Heise C. et al., (1997) Nature Medicine 3: 639-645; and Parr M. J. et al, (1997) Nature Medicine 3: 1145-1149). Several methods are available to deliver transgenes into a tumour *in vivo,* among which adenovirus- and lentivirus-based vectors (Benihoud K. et al., (1999) Curr. Opin. Biotechnol. 10: 440-447).

Thus there is need for alternative or improved systems for tumour cell-targeted therapies against cancers, in particular, liver cancer.

### SUMMARY OF THE INVENTION

The present invention relates to novel nucleic acids for expressing polynucleotides of interest specifically in mammalian cancer cells.

More specifically, this invention pertains to nucleic acids as defined above that comprise at least one polynucleotide of interest to be specifically expressed in cancer cells, which polynucleotide of interest is placed under the control of a so-called cancer cell-specific regulatory polynucleotide that drives its expression specifically in cancer cells. According to the present invention, the said regulatory polynucleotide is chosen from a group consisting of various selected polynucleotides that are derived from the promoter regions of the genes encoding human AFP, rat AFP, human HIP/PAPI and rat PAPI proteins.

According to the invention, the said polynucleotide of interest that is placed under the control of the said cancer cell-specific regulatory sequences may encode any molecule useful against cancer, including nucleic acids, e.g. RNA molecules, and proteins.

This invention also encompasses vectors comprising the said novel nucleic acids for expressing polynucleotides of interest specifically in mammalian cancer cells, as well as host cells that have been transfected or transduced with such vectors.

This invention also relates to pharmaceutical compositions comprising, as active ingredients, the said novel nucleic acids or said vectors.

### BRIEF DESCRIPTION OF THE FIGURES

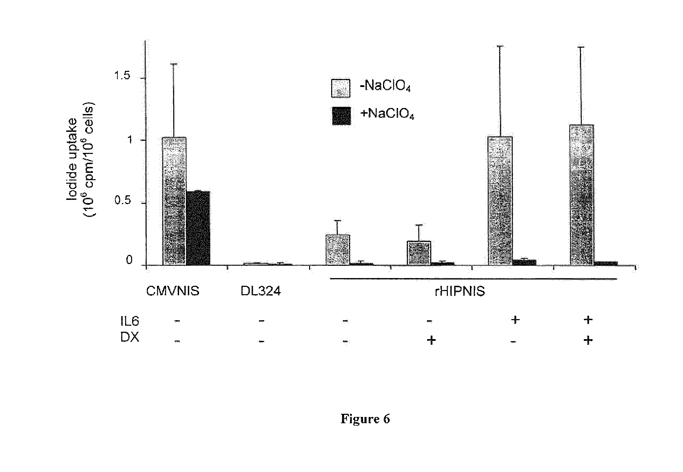

**Figure 1** depicts vector pShuttle-rAFPwt-rNIS
**Figure 2** depicts vector pShuttle-hAFPt-rNIS
**Figure 3** depicts vector pShuttle-hHIP/PAP-rNIS

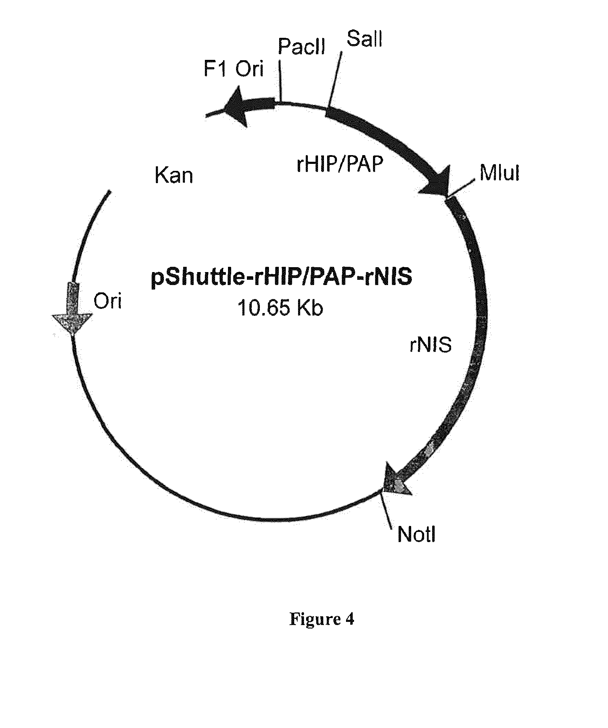

**Figure 4** depicts vector pShuttle-rHIP/PAP-rNIS
**Figure 5** illustrates the functional expression of NIS in tumour hepatocytes under the control of a rat HIP/PAP regulatory sequence.
   *In vitro* ¹²³I uptake in AdrHIPrNIS-infected HepG2 cells at different multiplicity of infection (MOI). NI: Non infected; DL324: Empty vector at MOI:10; 3; AdCMVrNIS at MOI:10 and 100; AdrHIPrNIS at MOI:25, 50, 75; 8 and 100 infectious particles. Each data point represents an average of three different experiments. -NaClO₄: without inhibition by sodium perchlorate. +NaClO₄: after inhibition by sodium perchlorate
**Figure 6** illustrates the stimulation of NIS expression in tumour hepatocytes under the control of a rat human HIP/PAP regulatory sequence after cell incubation with Interleukin 6 and/or Dexamethasone.
   ¹²³I uptake in AdrHIPNIS-infected HepG2 cells at MOI:100 under different conditions of stimulation as compared to uptake observed in AdCMVrNIS-infected cells. Each data point represents an average of three different experiments. -NaClO₄, without inhibition by sodium perchlorate. +NaClO₄ : after inhibition by sodium perchlorate.

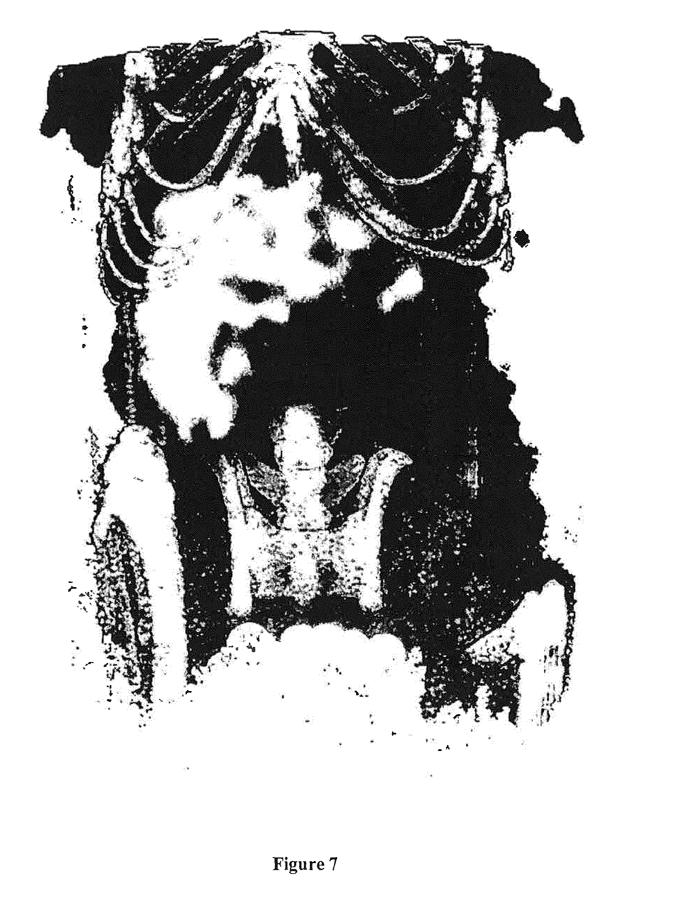

**Figure 7** illustrates a ⁹⁹Tc SPECT/CT imaging of a rat affected with a liver tumour and administered with a DNA construct comprising NIS sequence operably linked to a rat HIP/PAP regulatory sequence.
   ⁹⁹Tc SPECT/CT imaging of AdrHIPNIS-infected DEN rat. Strong hepatic contrast indicating high levels of NIS expression in transduced tumour nodules.

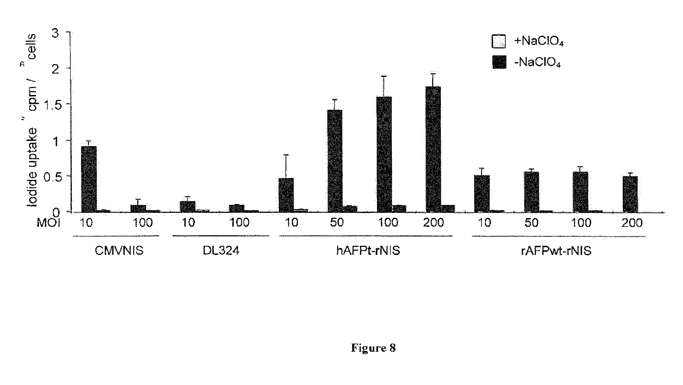

**Figure 8****_**illustrates the functional expression of NIS under the control of various AFP-derived regulatory sequences.
   *In vitro* ¹²³I uptake of HepG2 cells infected with AdhAFPt-rNIS and AdrAFPwt-rNIS at different multiplicity of infection. -NaClO₄: without inhibition by sodium perchlorate. +NaClO₄: after inhibition by sodium perchlorate. Each data point represents an average of three different experiments.

### DETAILED DESCRIPTION OF THE INVENTION

The present invention provides novel regulatory polynucleotides that drive the expression of polynucleotides of interest specifically in cancer cells.

Thus, the present invention provides new therapeutic tools that specifically target cancer cells in view of achieving the desired biological effect mainly, or even exclusively, at the target site, for a more efficient treatment. Simultaneously, the cancer cell-targeted therapeutic tools that have been newly designed lead to reduced, or even to none, side effects for the non-cancerous cells or organs of the body.

Surprisingly, it has been found according to the invention that a family of regulatory sequences derived from the promoter regions located upstream the genes encoding human and rat AFP, and human and rat HIP/PAPI proteins have the capability to direct expression of polynucleotides of interest specifically in cancer cells. Further, due to a high transcriptional activity of the regulatory sequences according to the invention, a strong expression of the said polynucleotides of interest that are operably linked therewith is obtained.

According to the invention, it has been constructed viral vectors combining (i) a polynucleotide of interest to be expressed in cancer cells, namely the sodium iodide symporter NIS with (ii) several different AFP, HIP/PAP regulatory sequences from different species. NIS protein expression and subcellular localization as well as NIS function have been evaluated in a series of human and murine, hepatic and non-hepatic, normal and transformed cells after infection by these vectors *in vitro,* and *in vivo* using several methods: immunoblot performed on purified membrane protein extracts; immunofluorescence; immunohistochemistry; radioiodine uptake and efflux assays; kinetic studies using serial scintigraphic and SPECT/CT imaging. The *in vivo* studies were performed in a chemically induced model of hepatocellular carcinoma (HCC) in Wistar rats. A very aggressive multinodular HCC sets in after 2 months of administration of the carcinogen diethylnitrosamine (DEN). It has been shown, according to the invention, that the NIS-expression pattern induced was strictly restricted to liver tumours *in vivo* for all the investigated vectors. NIS is strongly expressed at the plasma membrane of hepatoma transduced cells and permits radioiodine uptake levels comparable to, or even higher than, those obtained with a recombinant adenovirus, in which NIS is under the control of the ubiquitous strong cytomegalovirus promoter (AdCMV-NIS). No expression of NIS is found in infected primary hepatocytes and non-hepatic transformed cell lines. *In vivo,* the NIS transduction efficiency is high for the three routes of administration (portal vein, hepatic artery, intratumour) studied. The hepatic-artery route was more efficient than the portal-vein route due to the fact that the vascularisation of hepatic tumours occurs mostly through the branches of the hepatic artery. The intratumour and hepatic-artery routes led to a clear differential of NIS expression in favour of the tumour areas. About 30% of the injected radioiodide was concentrated in liver tumours, where its iodine biological half-life was of 2.5 days. Injection of 18 mCi of ¹³¹I led to the destruction of the irradiated transduced tumour nodules, while non-injected nodules continued to grow.

Furthermore, the inventors have also used the HIP/PAPI regulatory sequences described herein to target a protein of interest, specifically the NIS protein, to transformed cell lines derived from various cancerous organs, including human cancerous colon and human cancerous pancreas. In these transfected cancerous cell lines, a strong NIS expression at the plasma membrane was induced, and hence high levels of iodine uptake in these cellswere shown.

In summary, it is shown, according to the invention, that the new NIS recombinant constructs containing human or rat AFP-derived regulatory sequences, or containing human or rat HIP/PAPI-derived regulatory sequences, which are the object of this application, actually induce a targeted expression of a functional NIS in tumour hepatocytes, tumour colon cells and tumour pancreas cells, and that, moreover, this expression and taking-up activity are strong, namely, comparable, or even stronger than those induced by the ubiquitous CMV promoter.

### NUCLEIC ACIDS ACCORDING TO THE INVENTION

Are provided, according to the present invention, novel regulatory polynucleotides functional specifically in cancerous cells, which are described generally, and then in detail hereafter. These novel regulatory polynucleotides are used herein for expressing various polynucleotides of interest specifically in cancerous cells. As intended herein, the polynucleotides of interest encode especially nucleic acids or proteins that possess an anti-tumour effect.

Thus, an object of the invention consists of a nucleic acid for expressing a polynucleotide of interest specifically in mammalian cancer cells, the said nucleic acid comprising :
(a) a regulatory polynucleotide that drives the expression of the said polynucleotide of interest in mammalian cancer cells, which is selected from the group of regulatory polynucleotides consisting of :
   (i) a regulatory polynucleotide comprising, from 5'-end to 3'-end :
      - a first nucleic acid having at least 90% nucleotide identity with a nucleic acid selected from the group consisting of SEQ ID N°1 and SEQ ID N°2; and
      - a second nucleic acid having at least 90% nucleotide identity with the nucleic acid of SEQ ID N°3;
      - the said first and second nucleic acids being linked directly one to each other, or
      - the said first and second nucleic acids being linked indirectly one to each other through a spacer nucleic acid [having from 1 to 3000 nucleotides in length].
   (ii) a regulatory polynucleotide having at least 90% nucleotide identity with the nucleic acid starting at the nucleotide located at position 2200 and ending at the nucleotide located at position 2432 of SEQ ID N°4;
   (iii) a regulatory polynucleotide having at least 90% nucleotide identity with the nucleic acid starting at the nucleotide located at position 980 and ending at the nucleotide located at position 1253 of SEQ ID N°5; and
   (iv) a regulatory polynucleotide comprising, from 5'-end to 3'-end :
      - a first nucleic acid having at least 90% nucleotide identity with a nucleic acid selected from the group consisting of SEQ ID N°6 and SEQ ID N°7; and
      - a second nucleic acid having at least 90% nucleotide identity with the nucleic acid of SEQ ID N°8;
      - the said first and second nucleic acids being linked directly one to each other, or
      - the said first and second nucleic acids being linked indirectly one to each other through a spacer nucleic acid [having from 1 to 2000 nucleotides in length].
         and
(b) the said polynucleotide of interest.

As intended herein, a determined polynucleotide having at least 90% nucleotide identity with a reference polynucleotide possesses at least 90%, 91%, 92%, 93%, 94%, 95%, 96%, 97%, 98%, 99% or 99.5% nucleotide identity with said reference polynucleotide.

"Percentage of sequence identity," as used herein, is determined by comparing two optimally aligned sequences over a comparison window, where the fragment of the polynucleotide or amino acid sequence in the comparison window may comprise additions or deletions (e.g., gaps or overhangs) as compared to the reference sequence (which does not comprise additions or deletions) for optimal alignment of the two sequences. The percentage is calculated by (i) determining the number of positions at which the identical nucleic acid base or amino acid residue occurs in both sequences to yield the number of matched positions, (ii) dividing the number of matched positions by the total number of positions in the window of comparison and (iii) multiplying the result by 100 to yield the percentage of sequence identity. Optimal alignment of sequences for comparison may be conducted by the local homology algorithm of Smith and Waterman Add. Apl. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, Wis.), or by inspection. Given that two sequences have been identified for comparison, GAP and BESTFIT are preferably employed to determine their optimal alignment. Typically, the default values of 5.00 for gap weight and 0.30 for gap weight length are used.

For optimal comparison purposes, the percent of identity of two nucleic acid sequences can be achieved with CLUSTAL W (version 1.82) with the following parameters : (1) CPU MODE = ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = «default» ; (8) SCORE TYPE = « percent » ; (9) TOPDIAG = « default » ; (10) PAIRGAP = «default» ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = «cladogram» et (18) TREE GRAP DISTANCES = « hide ».

Are encompassed herein, as regulatory polynucleotides, any polynucleotide having at least 90% nucleic acid identity with a reference regulatory polynucleotide described above and that possesses the ability to drive the expression of a polynucleotide of interest specifically in a mammalian cancerous cell, including a human cancerous cell.

In a nucleic acid for expressing a polynucleotide of interest specifically in mammalian cancer cells, such as defined above, said regulatory polynucleotide (a) is « operably linked » to the said polynucleotide of interest.

As used herein, an "operable linkage" means a linkage in which the said regulatory polynucleotide is connected to the said polynucleotide of interest in such a way as to place transcription of the polynucleotide of interest under the influence of, or control of, the said regulatory polynucleotide. Two DNA sequences (such as a polynucleotide of interest to be transcribed and a regulatory polynucleotide linked to the 5' end of the polynucleotide of interest to be transcribed) are said to be operably linked, one to each other, if an induction of the regulatory polynucleotide function results in the transcription of mRNA encoding the polynucleotide of interest and if the nature of the linkage between the two DNA sequences does not (1) result in the introduction of a frame-shift mutation, (2) interfere with the ability of the regulatory polynucleotide to direct the expression of the protein, antisense RNA or ribozyme, or (3) interfere with the ability of the DNA template to be transcribed. Thus, a regulatory polynucleotide would be operably linked to a polynucleotide sequence of interest when the said regulatory polynucleotide is capable of effecting transcription of that polynucleotide sequence of interest.

Any of the regulatory polynucleotides that are operably linked to a polynucleotide of interest, in a nucleic acid according to the invention such as defined above, has the capability of directing transcription of the said polynucleotide of interest, specifically in cancerous cells. The specific activity of the above regulatory polynucleotides does not exclude that a low level of expression of the said polynucleotide of interest cannot occur also in normal non-cancerous cells. However, the regulatory polynucleotides that are described herein direct transcription of the said polynucleotide of interest highly preferentially, including exclusively, to mammalian cancerous cells. By "high preference" is meant an at least 3-fold, preferably 5-fold, more preferably at least 10-fold still more preferably at least 20-fold, 50-fold or 100-fold increase in transcript levels of said polynucleotide of interest in cancerous cells, as compared to the transcription level in normal non-cancerous cells.

As used herein, cancerous cells designates cells possessing characteristics typical of cancer-causing cells, such as uncontrolled proliferation, immortality, metastatic potential, rapid growth and proliferation rate, and certain specific morphological features. Cancerous cells are often in the form of a tumour, but such cells may exist alone within the body, or may be non-tumorigen cancer cells, such as a leukaemia cell. Cancerous cells can be associated with many kinds of cancers including, but not limited to leukemia, lymphoma, liver cancer, brain cancer, cerebrospinal cancer, bladder cancer, prostate cancer, breast cancer, cervix cancer, uterus cancer, ovarian cancer, kidney cancer, esophagus cancer, lung cancer, colon cancer, melanoma, neuroblastoma, and pancreatic cancer.

Preferably, cancerous cells according to the invention consist of cancerous cells from the liver, including cancerous hepatocytes.

In all cases, in a nucleic acid according to invention such as defined above, the polynucleotide of interest that is operably linked to the regulatory polynucleotide is "heterologous" with respect to the said regulatory polynucleotide. This means that the said polynucleotide of interest does not encompass a polynucleotide encoding human or rat AFP, nor human or rat HIP/PAPI proteins.

Preferred embodiments of the regulatory polynucleotides that are included in a nucleic acid for expressing a polynucleotide of interest specifically in mammalian cancer cells, such as defined above, are described hereafter.

### Preferred embodiments of regulatory polynucleotides derived from the human AFP promoter.

It has been shown according to the invention that the shortest functional regulatory polynucleotide derived from the promoter region of the gene encoding the human AFP protein, for specific expression of a polynucleotide of interest in mammalian cancer cells is a regulatory polynucleotide consisting of, from 5'-end to 3'-end :
- a first nucleic acid having at least 90% nucleotide identity with a nucleic acid selected from the group consisting of SEQ ID N°1 and SEQ ID N°2; and
- a second nucleic acid having at least 90% nucleotide identity with the nucleic acid of SEQ ID N°3,
the nucleotide located at the 3'-end of the said first nucleic acid being covalently bound to the nucleotide located at the 5'-end of the said second nucleic acid.

The promoter region of the gene encoding human AFP protein was already known in the art. The minimal functional sequences from the human AFP gene promoter were disclosed by Watanabe et al. (1987, J. Biol., Chem., Vol. 282(10) : 4812-4818). According to Watanabe et al. (1987), the sequences that are important for enhancer activity reside in the 400-base pair region between 3.3 and 3.7 kilobase pairs upstream of the AFP gene. Using CAT constructs bearing AFP 5'-flanking sequences, Watanabe et al. (1987) have shown that no transcription of the CAT-encoding sequence occurred with shorter AFP promoter fragments that lacked the distal enhancer elements located 3.7 kilobase-pairs upstream of the AFP gene. Notably, no CAT expression could be detected with AFP promoter fragments comprising only 2.9 kilobase-pairs upstream of the AFP gene.

Very surprisingly, it has been found according to the invention that human AFP promoter fragments as described above, that comprise not more than 1000 nucleotides in length successfully direct transcription of a polynucleotide of interest, specifically in cancerous cells,

Further, it is shown herein that regulatory sequences comprising, from 5'-end to 3'-end, the said first nucleic acid having at least 90% nucleotide identity with SEQ ID N° 1 (or SEQ ID N° 2) and the said second nucleic acid having at least 90% nucleotide identity with SEQ ID N° 3, and wherein the said first and second nucleic acids are separated by a spacer nucleic acid possess also the ability to direct transcription of a polynucleotide of interest, specifically in cancerous cells.

The nucleic acids of SEQ ID N°1 and SEQ ID N° 2, being in sense orientation and antisense orientation respectively, are believed herein to impart the specificity of expression in mammalian cancer cells, whereas the nucleic acid of SEQ ID N° 3 is believed to consist of the minimal sequence endowed with a promoter function.

Consequently, functional regulatory polynucleotides of interest according to the invention encompass those that comprise, from 5'-end to 3'end :
- a first nucleic acid having at least 90% nucleotide identity with a nucleic acid selected from the group consisting of SEQ ID N°1 and SEQ ID N°2; and
- a second nucleic acid having at least 90% nucleotide identity with the nucleic acid of SEQ ID N°3;
- the said first and second nucleic acids being linked directly one to each other, or
- the said first and second nucleic acids being linked indirectly one to each other through a spacer nucleic acid [having from 1 to 3000 nucleotides in length].

An embodiment of such a regulatory polynucleotide wherein the said first and second nucleic acids are directly linked one to each other is illustrated herein by the regulatory polynucleotide of SEQ ID N° 9, comprising, from 5'-end to 3'-end, the nucleic acid of SEQ ID N°2 which is directly linked to the nucleic acid of SEQ ID N°3.

Another embodiment of such a regulatory sequence wherein the said first and second nucleic acids are linked indirectly one to each other through a spacer nucleic acid is illustrated herein by the regulatory polynucleotide of SEQ ID N° 10 comprising, from 5'-end to 3'-end, the nucleic acid of SEQ ID N°1, the said first nucleic acid being linked through its 3-end to a spacer nucleic acid having 2835 nucleotides in length, and the said spacer nucleotide being linked through its 3'-end to the said second nucleic acid of SEQ ID N°3.

Generally, the spacer nucleic acid, if it is present in the regulatory sequence, has a nucleotide length ranging from 1 to 3000 nucleotides, each nucleotide being preferably selected from the group consisting of Adenosine (also termed "A"), Thymidine (also termed "T"), Guanosine (also termed "G"), Cytosine (also termed "C") and in some cases Uridine (also termed "U").

In certain embodiments, the spacer nucleic acid has up to 2, 3, 4, 5, 6, 7, 8, 9, 10, 20, 30, 40, 50, 60, 70, 80, 90, 100, 150, 200, 250, 300 , 350, 400, 1450, 500, 1550, 600, 650, 700, 750, 800, 850, 900, 950, 1000, 1100, 1200, 1300 , 1400, 1500, 1550, 1600, 1650, 1700, 1750, 1800, 1850, 1900, 1950, 2000, 2050, 2100, 2150, 2200, 2250, 2300 , 2350, 2400, 2450, 2500, 2550, 2600, 2650, 2700, 2750, 2800, 2850, 2900, 2950 nucleotides in length.

In certain embodiments, the spacer nucleic acid consists of consecutive nucleotides from the human AFP promoter, for example from at least 2 to at least 2500 consecutive nucleotides from the human AFP promoter like in the regulatory sequence of SEQ ID N°10 herein.

Thus, are encompassed herein regulatory polynucleotides consisting of :
- a first nucleic acid having at least 90% nucleotide identity with a nucleic acid selected from the group consisting of SEQ ID N°1 and SEQ ID N°2;
- a spacer nucleic acid having a nucleotide length ranging from 1 to 3000 nucleotides, and
- a second nucleic acid having at least 90% nucleotide identity with the nucleic acid of SEQ ID N°3;

Indeed, a regulatory sequence derived from the human AFP promoter according to the present invention may also comprise :
- a 5'-additional nucleic acid that is linked to the 5'-end of the said first nucleic acid, which may also be termed herein a 5'-flanking sequence; and/or
- a 3'-additional nucleic acid that is linked to the 3'-end of the said second nucleic acid, which may also be termed herein a 3'-flanking nucleic acid.

The said 5'-additional nucleic acid and the said 3'-additional nucleic acid have a nucleotide length ranging from 1 to 1500 nucleotides, preferably from 1 to 1500 nucleotides.

Notably, the said 5'-additional nucleic acid and the said 3'-additional nucleic acid may consist of restriction site sequences recognised by nucleases, including endonucleases, e.g. for purpose of cloning in suitable nucleic acid constructs such as cloning vectors or expression vectors.

In certain embodiments, the said 5'-additional nucleic acid and/or the said 3'-additional nucleic acid have a nucleotide length of at least 2, 3, 4, 5, 6, 7, 8, 9, 10, 20, 30, 40, 50, 60, 70, 80, 90, 100, 150, 200, 250, 300 , 350, 400, 1450, 500, 1550, 600, 650, 700, 750, 800, 850, 900, 950, 1000, 1100, 1200, 1300 or 1400. Illustratively, the regulatory polynucleotide of SEQ ID N° 10 comprises a 5'-additional polynucleotide of 604 nucleotides in length.

The present invention encompasses a regulatory polynucleotide having at least 90% nucleotide identity with the nucleic acid of SEQ ID N°9, and thus encompasses the nucleic acid of SEQ ID N° 9 itself.

The present invention encompasses a regulatory polynucleotide having at least 90% nucleotide identity with the nucleic acid of SEQ ID N° 10, and thus encompasses the nucleic acid of SEQ ID N° 10 itself.

### Preferred embodiments of regulatory polynucleotides derived from the human HIP/PAPI promoter.

It has been shown according to the invention that the shorter functional regulatory polynucleotide derived from the promoter region of the gene encoding the human HIP/PAPI protein, for specific expression of a polynucleotide of interest in mammalian cancer cells, consists of the regulatory polynucleotide starting at the nucleotide located at position 2200 and ending at the nucleotide located at position 2432 of SEQ ID N°4, which is also the regulatory polynucleotide of SEQ ID N° 11 herein.;

HIP/PAP protein is a C-type lectin overexpressed in hepatocellular carcinoma (HCC). Pleiotropic biological activities have been ascribed to this protein, but little was known about the function of HIP/PAP in the liver. As disclosed in the PCT application n° WO 2004/112824, HIP/PAPI was shown to possess mitogenic and antiapoptotic effects *in vitro* on hepatocytes in primary cell culture. Also, HIP/PAP was a mitogenic and antiapoptotic molecule for hepatocytes, *in vivo,* during liver failure and liver regeneration. PCT application n° WO 2004/11824 also disclosed DNA constructs comprising the HIP/PAPI coding sequence operably linked (i) to the rabbit WAP gene promoter or (ii) to the mouse albumin gene 18 promoter.

The promoter region of the gene encoding human HIP/PAPI protein has not been functionally studied in the prior art.

According to the present invention, it has now been shown that the sequence SEQ N° 4, which is, in the human genome, located upstream the sequence encoding the HIP/PAPI protein, consists of a regulatory sequence possessing the ability to direct transcription of a polynucleotide of interest that is operably linked thereto, specifically in mammalian cancer cells.

It has further been shown that various nucleic acid fragments obtained by cleavage of the nucleic acid of SEQ ID N° 4 also possess the ability to direct transcription of a polynucleotide of interest that is operably linked thereto, specifically in mammalian cancer cells, which nucleic acid fragments all comprise the nucleic acid of SEQ ID N° 11.

Still further, it has been shown herein that the nucleic acid of SEQ ID N° 11 itself possesses the ability to direct transcription of a polynucleotide of interest that is operably linked thereto, specifically in mammalian cancer cells.

Consequently, functional regulatory polynucleotides of interest according to the invention encompass those that comprise a nucleic acid of SEQ ID N° 11.

Those regulatory polynucleotides of interest encompass the various functional fragments of the HIP/PAPI promoter that are disclosed in the examples herein, including the nucleic acids of SEQ ID N° 12, SEQ ID N° 13 and SEQ ID N°14.

Thus, within a nucleic acid for expressing a polynucleotide of interest specifically in mammalian cancer cells, said regulatory polynucleotide may be selected from the group consisting of :
- a regulatory polynucleotide having at least 90% nucleotide identity with the nucleic acid comprising SEQ ID N° 11;
- a regulatory polynucleotide having at least 90% nucleotide identity with the nucleic acid comprising SEQ ID N° 12;
- a regulatory polynucleotide having at least 90% nucleotide identity with the nucleic acid comprising SEQ ID N° 13;
- a regulatory polynucleotide having at least 90% nucleotide identity with the nucleic acid comprising SEQ ID N° 14; and
- a regulatory polynucleotide having at least 90% nucleotide identity with the nucleic acid comprising SEQ ID N° 4;

Further, within a nucleic acid for expressing a polynucleotide of interest specifically in mammalian cancer cells, said regulatory polynucleotide may be selected from the group consisting of :
- a regulatory polynucleotide having at least 90% nucleotide identity with the nucleic acid consisting of SEQ ID N° 11;
- a regulatory polynucleotide having at least 90% nucleotide identity with the nucleic acid consisting of SEQ ID N° 12;
- a regulatory polynucleotide having at least 90% nucleotide identity with the nucleic acid consisting of SEQ ID N° 13;
- a regulatory polynucleotide having at least 90% nucleotide identity with the nucleic acid consisting of SEQ ID N° 14;and
- a regulatory polynucleotide having at least 90% nucleotide identity with the nucleic acid consisting of SEQ ID N° 4.

Generally, within a nucleic acid for expressing a polynucleotide of interest specifically in mammalian cancer cells, said regulatory polynucleotide possesses at least 233 consecutive nucleotides of the nucleic acid of SEQ ID N° 4 and comprises the nucleic acid of SEQ ID N° 11. Illustratively, the regulatory polynucleotide of SEQ ID N° 12 comprises 573 nucleotides consecutive nucleotides of SEQ ID N° 4 and comprises SEQ ID N° 11. Still illustratively, the regulatory polynucleotide of SEQ ID N° 13 comprises 1332 consecutive nucleotides of SEQ ID N°4 and comprises SEQ ID N° 11. Yet illustratively, the regulatory polynucleotide of SEQ ID N° 14 comprises 2056 consecutive nucleotides of SEQ ID N° 4 and comprises SEQ ID N° 11.

Such a regulatory polynucleotide may comprise up to 2426 consecutive nucleotides in length of SEQ ID N° 4. Such regulatory polynucleotides may thus comprise at least 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 410, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1100, 1110, 1120, 1130, 1140, 1150, 1160, 1170, 1180, 1190, 1200, 1210, 1220, 1230, 1240, 1250, 1260, 1270, 1280, 1290, 1300, 1310, 1320, 1330, 1340, 1350, 1360, 1370, 1380, 1390, 1400, 1410, 1420, 1430, 1440, 1450, 1460, 1470, 1480, 1490, 1500, 1510, 1520, 1530, 1540, 1550, 1560, 1570, 1580, 1590, 1600, 1610, 1620, 1630, 1640, 1650, 1660, 1670, 1680, 1690, 1700, 1710, 1720, 1730, 1740, 1750, 1760, 1770, 1780, 1790, 1800, 1810, 1820, 1830, 1840, 1850, 1860, 1870, 1880, 1890, 1900, 1910, 1920, 1930, 1940, 1950, 1960, 1970, 1980, 1990, 2000, 2010, 2020, 2030, 2040, 2050, 2060, 2070, 2080, 2090, 2100, 2110, 2120, 2130, 2140, 2150, 2160, 2170, 2180, 2190, 2200, 2210, 2220, 2230, 2240, 2250, 2260, 2270, 2280, 2290, 2300, 2310, 2320, 2330, 2340, 2350, 2360, 2370, 2380, 2390, 2400, 2410 or 2420 consecutive nucleotides of SEQ ID N°4 and comprises the nucleic acid of SEQ ID N° 11.

Are also encompassed regulatory polynucleotides having at least 90% nucleotide identity with the regulatory polynucleotides defined above.

Indeed, a regulatory sequence derived from the human HIP/PAPI promoter according to the present invention may also comprise :
- a 5'-additional nucleic acid that is linked to the 5'-end of the said first nucleic acid, which may also be termed herein a 5'-flanking sequence; and/or
- a 3'-additional nucleic acid that is linked to the 3'-end of the said second nucleic acid, which may also be termed herein a 3'-flanking nucleic acid.

The said 5'-additional nucleic acid and the said 3'-additional nucleic acid have a nucleotide length ranging from 1 to 2500 nucleotides, preferably from 1 to 1500 nucleotides.

Notably, the said 5'-additional nucleic acid and the said 3'-additional nucleic acid may consist of restriction site sequences recognised by nucleases, including endonucleases, e.g. for purpose of cloning in suitable nucleic acid constructs such as cloning vectors or expression vectors.

In certain embodiments, the said 5'-additional nucleic acid and/or the said 3'-additional nucleic acid have a nucleotide length of at least 2, 3, 4, 5, 6, 7, 8, 9, 10, 20, 30, 40, 50, 60, 70, 80, 90, 100, 150, 200, 250, 300 , 350, 400, 1450, 500, 1550, 600, 650, 700, 750, 800, 850, 900, 950, 1000, 1100, 1200, 1300 or 1400. Illustratively, the regulatory polynucleotide of SEQ ID N° 4 comprises a 5'-additional polynucleotide of more than 2200 nucleotides in length, upstream of the regulatory polynucleotide of SEQ ID N° 11.

### Preferred embodiments of regulatory polynucleotides derived from the rat HIP/PAPI promoter.

It has been shown according to the invention that regulatory polynucleotides derived from the rat HIP/PAPI promoter are able to direct the expression of a polynucleotide of interest specifically in mammalian cancer cells.

Notably it is shown herein that the nucleic acid of SEQ ID N° 5 is able to direct the expression of a polynucleotide of interest specifically in cancer cells.

The rat HIP/PAPI gene was known in the art to undergo more than a 200-fold expression increase during the acute phase of pancreatitis (1991, lovanna et al., J Biol Chem, Vol. 266 : 24664-24669).

The rat HIP/PAPI gene promoter region was already known *per se* in the prior art. Using pancreatic cells transfected with DNA constructs bearing various deletion fragments of the rat HIP/PAPI promoter fused to the CAT gene, it has been shown in the art that several of the promoter fragments tested were able to induce the expression of the CAT reporter gene, when the transfected cells were incubated with a mixture of IL-6 and dexamethasone (1995, Dusetti et al., J Biol Chem, Vol. 270(38) : 22417-22421). Due to the sensitivity of the rat HIP/PAPI promoter to induction by a mixture of IL-6 and dexamethasone, Dusetti et al. (1995) stated that the rat HIP/PAPI gene should be classified among acute phase proteins of class 2, whose expression is increased by IL-6 acting in combination with glucocorticoids.

Thus, according to the present invention it has now been shown that the sequence SEQ ID N° 5, which is, in the rat genome, located upstream the sequence encoding the rat HIP/PAPI protein, consists of a regulatory sequence possessing the ability to direct transcription of a polynucleotide of interest that is operably linked thereto, specifically in mammalian cancer cells.

Without wishing to be bound by any particular theory, it is believed that any one of the nucleic acid fragments of the rat HIP/PAPI promoter that have been shown earlier to be functional in pancreatic cells under specific induction conditions are also functional for expressing a polynucleotide of interest specifically in mammalian cancer cells.

Thus, within a nucleic acid for expressing a polynucleotide of interest specifically in mammalian cancer cells according to the invention, the said regulatory polynucleotide may also be selected from the group of regulatory polynucleotides comprising the shortest functional nucleic acid fragment derived from the rat HIP/PAPI promoter, or nucleic acid fragments having .a strong nucleotide identity therewith.

Thus, another object of the invention consists of a nucleic acid for expressing a polynucleotide of interest specifically in mammalian cancer cells as defined generally above and wherein the regulatory polynucleotide included therein consists of a nucleic acid having at least 90% nucleotide identity with the nucleic acid starting at the nucleotide in position 980 and ending at the nucleotide in position 1253 of SEQ ID N°5, the said nucleic acid being the shortest nucleic acid fragment derived from the rat HIP/PAPI promoter that was earlier shown to be functional in pancreatic cells under specific induction conditions.

Thus, those regulatory polynucleotides encompass nucleic acids comprising at least 274 consecutive nucleotides of the nucleic acid of SEQ ID N° 5 and which comprise the nucleic acid starting at the nucleotide in position 980 and ending at the nucleotide in position 1253 of SEQ ID N°5. Illustratively, those regulatory polynucleotides encompass nucleic acids comprising 317 consecutive nucleotides of the nucleic acid of SEQ ID N° 5 and which comprise the nucleic acid starting at the nucleotide in position 980 and ending at the nucleotide in position 1253 of SEQ ID N°5. Further illustratively, those regulatory polynucleotides encompass nucleic acids comprising 379 consecutive nucleotides of the nucleic acid of SEQ ID N° 5 and which comprise the nucleic acid starting at the nucleotide in position 980 and ending at the nucleotide in position 1253 of SEQ ID N°5. Yet illustratively, those regulatory polynucleotides encompass nucleic acids comprising 444 consecutive nucleotides of the nucleic acid of SEQ ID N° 5 and which comprises the nucleic acid starting at the nucleotide in position 980 and ending at the nucleotide in position 1253 of SEQ ID N°5. within a nucleic acid for expressing a polynucleotide of interest specifically in mammalian cancer cells, are encompassed herein regulatory polynucleotides having at least 90% nucleotide identity with a nucleic acid comprising at least 274, 275, 276, 277, 278, 279, 280, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 410, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1100, 1110, 1120, 1130, 1140, 1150, 1160, 1170, 1180, 1190, 1200, 1210, 1220, 1230, 1240 or 1250 consecutive nucleotides of SEQ ID N° 5 and which comprises the nucleic acid starting at the nucleotide in position 980 and ending at the nucleotide in position 1253 of SEQ ID N°5

Indeed, a regulatory sequence derived from the human HIP/PAPI promoter according to the present invention may also comprise :
- a 5'-additional nucleic acid that is linked to the 5'-end of the said first nucleic acid, which may also be termed herein a 5'-flanking sequence; and/or
- a 3'-additional nucleic acid that is linked to the 3'-end of the said second nucleic acid, which may also be termed herein a 3'-flanking nucleic acid.

The said 5'-additional nucleic acid and the said 3'-additional nucleic acid have a nucleotide length ranging from 1 to 2500 nucleotides, preferably from 1 to 1500 nucleotides.

Notably, the said 5'-additional nucleic acid and the said 3'-additional nucleic acid may consist of restriction site sequences recognised by nucleases, including endonucleases, e.g. for purpose of cloning in suitable nucleic acid constructs such as cloning vectors or expression vectors.

In certain embodiments, the said 5'-additional nucleic acid and/or the said 3'-additional nucleic acid have a nucleotide length of at least 2, 3, 4, 5, 6, 7, 8, 9, 10, 20, 30, 40, 50, 60, 70, 80, 90, 100, 150, 200, 250, 300 , 350, 400, 1450, 500, 1550, 600, 650, 700, 750, 800, 850, 900, or 950 nucleotides. Illustratively, the regulatory polynucleotide of SEQ ID N° 5 comprises a 5'-additional polynucleotide of more than 950 nucleotides in length, upstream of the regulatory polynucleotide starting at the nucleotide in position 980 and ending at the nucleotide in position 1253 of SEQ ID N°5.

### Preferred embodiments of regulatory polynucleotides derived from the rat AFP promoter.

It has been shown according to the invention that the shortest functional regulatory polynucleotide derived from the promoter region of the gene encoding the rat AFP protein, for specific expression of a polynucleotide of interest in mammalian cancer cells is a regulatory polynucleotide consisting of, from 5'-end to 3'-end :
- a first nucleic acid having at least 90% nucleotide identity with a nucleic acid selected from the group consisting of SEQ ID N°6 and SEQ ID N°7; and
- a second nucleic acid having at least 90% nucleotide identity with the nucleic acid of SEQ ID N°8,
the nucleotide located at the 3'-end of the said first nucleic acid being covalently bound to the nucleotide located at the 5'-end of the said second nucleic acid.

Very surprisingly, it has been found according to the invention that rat AFP promoter fragments as described above, that comprise not more than 1100 nucleotides in length, successfully direct transcription of a polynucleotide of interest, specifically in cancerous cells,

Further, it is shown herein that regulatory sequences comprising, from 5'-end to 3'-end, the said first nucleic acid having at least 90% nucleotide identity with SEQ ID N° 6 (or SEQ ID N° 7) and the said second nucleic acid having at least 90% nucleotide identity with SEQ ID N° 8, and wherein the said first and second nucleic acids are separated by a spacer nucleic acid possess also the ability to direct transcription of a polynucleotide of interest, specifically in cancerous cells.

The nucleic acids of SEQ ID N°6 and SEQ ID N° 7, being in sense orientation and antisense orientation respectively, are believed herein to impart the specificity of expression in mammalian cancer cells, whereas the nucleic acid of SEQ ID N° 8 is believed to consist of the minimal sequence endowed with a promoter function.

Consequently, functional regulatory polynucleotides of interest according to the invention encompass those that comprise, from 5'-end to 3'end :
- a first nucleic acid having at least 90% nucleotide identity with a nucleic acid selected from the group consisting of SEQ ID N°6 and SEQ ID N°7; and
- a second nucleic acid having at least 90% nucleotide identity with the nucleic acid of SEQ ID N°8;
- the said first and second nucleic acids being linked directly one to each other, or
- the said first and second nucleic acids being linked indirectly one to each other through a spacer nucleic acid [having from 1 to 2000 nucleotides in length].

An embodiment of such a regulatory sequence wherein the said first and second nucleic acids are linked indirectly one to each other through a small spacer nucleic acid is illustrated herein by the regulatory polynucleotide of SEQ ID N° 15 comprising, from 5'-end to 3'-end, the nucleic acid of SEQ ID N°7, the said first nucleic acid being linked through its 3-end to a spacer nucleic acid having 106 nucleotides in length, and the said spacer nucleotide being linked through its 3'-end to the said second nucleic acid of SEQ ID N°8.

Another embodiment of such a regulatory sequence wherein the said first and second nucleic acids are linked indirectly one to each other through a long spacer nucleic acid is illustrated herein by the regulatory polynucleotide of SEQ ID N° 16 comprising, from 5'-end to 3'-end, the nucleic acid of SEQ ID N°6, the said first nucleic acid being linked through its 3-end to a spacer nucleic acid having 1857 nucleotides in length, and the said spacer nucleotide being linked through its 3'-end to the said second nucleic acid of SEQ ID N°8.

Generally, the spacer nucleic acid, if it is present in the regulatory sequence, has a nucleotide length ranging from 1 to 2000 nucleotides, each nucleotide being preferably selected from the group consisting of Adenosine (also termed "A"), Thymidine (also termed "T"), Guanosine (also termed "G"), Cytosine (also termed "C") and in some cases Uridine (also termed "U").

Thus, are encompassed herein the regulatory polynucleotides consisting of :
- a first nucleic acid having at least 90% nucleotide identity with a nucleic acid selected from the group consisting of SEQ ID N°6 and SEQ ID N°7;
- a spacer nucleic acid having a nucleotide length ranging from 1 to 2000 nucleotides, and
- a second nucleic acid having at least 90% nucleotide identity with the nucleic acid of SEQ ID N°8;

In certain embodiments, the spacer nucleic acid has up to 2, 3, 4, 5, 6, 7, 8, 9, 10, 20, 30, 40, 50, 60, 70, 80, 90, 100, 150, 200, 250, 300 , 350, 400, 1450, 500, 1550, 600, 650, 700, 750, 800, 850, 900, 950, 1000, 1100, 1200, 1300 , 1400, 1500, 1550, 1600, 1650, 1700, 1750, 1800, 1850, 1900, 1950 or 2000 nucleotides in length.

In certain embodiments, the spacer nucleic acid consists of consecutive nucleotides from the rat AFP promoter, for example from at least 2 to at least 1800 consecutive nucleotides from the rat AFP promoter like in the regulatory sequence of SEQ ID N°16 herein.

Indeed, a regulatory sequence derived from the rat AFP promoter according to the present invention may also comprise :
- a 5'-additional nucleic acid that is linked to the 5'-end of the said first nucleic acid, which may also be termed herein a 5'-flanking sequence; and/or
- a 3'-additional nucleic acid that is linked to the 3'-end of the said second nucleic acid, which may also be termed herein a 3'-flanking nucleic acid.

The said 5'-additional nucleic acid and the said 3'-additional nucleic acid have a nucleotide length ranging from 1 to 1500 nucleotides, preferably from 1 to 1500 nucleotides.

Notably, the said 5'-additional nucleic acid and the said 3'-additional nucleic acid may consist of restriction site sequences recognised by nucleases, including endonucleases, e.g. for purpose of cloning in suitable nucleic acid constructs such as cloning vectors or expression vectors.

In certain embodiments, the said 5'-additional nucleic acid and/or the said 3'-additional nucleic acid have a nucleotide length of at least 2, 3, 4, 5, 6, 7, 8, 9, 10, 20, 30, 40, 50, 60, 70, 80, 90, 100, 150, 200, 250, 300 , 350, 400, 1450, 500, 1550, 600, 650, 700, 750, 800, 850, 900, 950, 1000, 1100, 1200, 1300 or 1400. Illustratively, the regulatory polynucleotide of SEQ ID N° 16 comprises a 5'-additional polynucleotide of 346 nucleotides in length, upstream of SEQ ID N° 6 that is comprised therein. Still illustratively, the regulatory polynucleotide of SEQ ID N° 16 comprises a 3'-additional polynucleotide of 76 nucleotides in length, downstream of SEQ ID N° 8 that is comprised therein.

The present invention encompasses a regulatory polynucleotide having at least 90% nucleotide identity with the nucleic acid of SEQ ID N°15, and thus encompasses the nucleic acid of SEQ ID N° 15 itself.

The present invention encompasses a regulatory polynucleotide having at least 90% nucleotide identity with the nucleic acid of SEQ ID N° 16, and thus encompasses the nucleic acid of SEQ ID N° 16 itself.

### Preferred polynucleotides of interest that are comprised in a nucleic acid for expressing the same specifically in mammalian cancer cells.

Within a nucleic acid for strongly expressing a polynucleotide of interest specifically in mammalian cells, such as defined in the present specification, said polynucleotide of interest may be of any kind, provided it is useful for prophylactic or therapeutic purpose against cancer.

In other words, said polynucleotide of interest, when specifically expressed in cancer cells under the control of said regulatory polynucleotide, should in all cases induce anti-cancer activity at a cellular level and/or at a tissue level and/or at an organ level and/or more systemically at the level of the whole mammalian organism body.

A wide variety of polynucleotides that possess anti-cancer activity that are known in the art may be used as polynucleotides of interest in a nucleic acid according to the invention.

Anticancer activity may be defined as the destruction and/or inhibition of proliferation and/or promotion of differentiation of cancer cells. Cancer cells are malignantly transformed cells known to those skilled in the art as cells with known and unknown abnormalities in growth regulatory genes and pathways. Such cells possess the capability of growing in an unregulated manner and may give rise to tumour formation in naturally occurring disease conditions or under experimental conditions. A tumor is defined as a homogenous or heterogeneous mass of cancer cells. Anticancer activity includes destruction and/or inhibition of proliferation and/or promotion of differentiation of cancer cells grown in vitro or cancer cells in a subject, either a human or a non-human animal. Destruction and/or inhibition of proliferation and/or promotion of differentiation of cancer cells may involve mechanisms known to those skilled in the art including but not limited to various pathways of differentiation, apoptosis (programmed cell death) or necrosis. Anticancer activity may further involve destruction and/or inhibition of proliferation and/or promotion of differentiation of disseminated cancer cells also known as metastatic cancer cells that have the capability of moving away form the site of an initial tumour and may be found at one, or more, distant sites from the originating tumour. Anticancer activity may further encompass reduction, complete dissolution, or inhibition of growth of localized or disseminated tumors comprising homogenous or heterogeneous populations of cancer cells.

Are encompassed by those polynucleotides that possess anti-cancer activity, polynucleotides which, when they are expressed in a cell, induce, directly or indirectly, cell death. These polynucleotides of interest are termed herein "suicide polynucleotides".

Suicide polynucleotides include :
(i) polynucleotides encoding proteins that are cytotoxic;
(ii) polynucleotides encoding proteins that induce cytotoxicity, when a further substance is present;
(iii) polynucleotides encoding RNAi that alter or block the expression of proteins required for cell viability, or to silence oncogenes;;
(iv) polynucleotides encoding cytotoxic ribonucleases;
(v) polynucleotides encoding ribozymes that target expression of proteins required for anti-cancer activity;
(vi) polynucleotides encoding sense or antisense nucleic acids that alter or block the expression of proteins required for anti-cancer activity;

### Polynucleotides of interest encoding anti-cancer genes

Illustrative examples of polynucleotides of interest having an anti-cancer effect, include genes with anti-proliferative activity, anti-metastatic activity, anti-angiogenic activity, or pro-apoptotic activity, such as mda-7/IL-24 (Sarkar et al. (2002) Biotechniques Suppl: 30-39; Fisher et al. (2003) Cancer Biol Ther 2:S23-37), TNF-alpha. (Anderson et al. Curr Opin Pharmacol (2004) 4 (4):314-320), IFN-beta. (Yoshida et al, (2004) Cancer Sci 95 (11):858-865), p53 (Haupt et al. Cell Cycle (2004) 3 (7):912-916), BAX (Chan et al. Clin Exp Pharmacol Physiol (2004) 31 (3):119-128), PTEN (Sansal et al. J Clin Oncol (2004) 22 (14):2954-63), soluble fibroblast growth factor receptor (sFGFR) (Gowardhan et al. (2004) Prostate 61 (1):50-59), RNAi or antisense-ras (Liu et al. Cancer Gene Ther (2004) 11 (11):748-756.), RNAi or antisense VEGF (Qui et al. Hepatobiliary Pancreat Dis Int (2004) 3 (4):552-557), antisense or RNAi mda-9/syntenin (Sarkar et al. Pharmacol Ther (2004) 104 (2):101-115).

Further illustrative examples of polynucleotides of interest having an anti-cancer effect include those that enhance radiotherapy including, but not limited to, p53 (Haupt et al. Cell Cycle (2004) 3 (7):912-916), GADD34 (Leibermann et al. Leukemia (2002) 16 (4):527-41), the sodium iodide symporter (for thyroid cancer) (Mitrofanova et al. Clin Cancer Res (2004) 10 (20):6969-6976) or the Na⁺/K⁺ ATPase (PCT application n° WO 98/45443).

Still further illustrative examples of polynucleotides of interest having an anti-cancer effect include those encoding the herpes simplex virus type-1 thymidine kinase gene (HTK), given in combination with the drug ganciclovir (GCV). HTK is the most commonly used cancer gene therapy system to date, both in experimental models and clinical trials. See J. Gomez-Navarro et al., "Gene therapy for cancer," European Journal of Cancer, vol. 35, pp. 867-885 (1999). HTK, whose substrate specificity is distinct from that of cellular thymidine kinases, can convert GCV to the toxic phosphorylated form, specifically killing the cells that express HTK. Since the concept of an HTK/GCV system was first described, it has shown good success as a tumour ablation strategy in a variety of experimental models. In addition, over two dozen clinical gene therapy trials based on this model have been initiated in the last seven years. See J. A. Roth et al., "Gene therapy for cancer: what have we done and where are we going?" Journal of the National Cancer Institute, vol. 89(1), pp. 21-39 (1997); D. Klatzmann et al., "A Phase I/II dose-escalation study of herpes simplex virus type 1 thymidine kinase "suicide" gene therapy for recurrent metastatic melanoma," Human Gene Therapy, vol. 9, pp. 2585-2894 (1998); and J. R. Herman et al.," In situ gene therapy for adenocarcinoma of the prostate: A phase I clinical trial," Human Gene Therapy, vol. 10, pp. 1239-1249 (1999).

### Polynucleotides of interest encoding RNAi or siRNA

Yet further illustrative embodiments of polynucleotides of interest having an anti-cancer effect include those encoding siRNAs that may be used to silence genes of interest to effect anti-cancer activity, including genes required for cell viability and oncogenes (see (i) Borkhardt, A., Blocking oncogenes in malignant cells by RNA interference : New hope for a highly specific cancer treatment? Cancer Cell, 2002. 2(3): p. 167-8 ; (ii) Wilda, M., et al., Killing of leukemic cells with a BCR/ABL fusion gene by RNA interference (RNAi). Oncogene, 2002. 21(37): p. 5716-24 ; (iii) Brummelkamp, T. R., R. Bernards, and R. Agami, Stable suppression of tumourigenicity by virus-mediated RNA interference. Cancer Cell, 2002. 2(3): p. 243-7).

For the purposes of this invention, the term "RNA interference" or "RNAi" is broadly defined and includes all post-transcriptional and transcriptional mechanisms of RNA mediated inhibition of gene expression, such as those described in P. D. Zamore Science 296, 1265 (2002). RNA interference is mediated by double-stranded RNA (dsRNA), which can induce many different epigenetic gene-silencing processes in eukaryotes, including the degradation of homologous mRNAs -a process called RNA interference (RNAi) in animals, and post-transcriptional gene silencing (PTGS) in plants. RNA interference (RNAi) was first discovered in 1998 by Andrew Fire and Craig Mello in C. elegans. Double stranded RNA has been shown to inhibit expression of genes having a complementary sequence through a process termed RNA interference (see Hammond et al. Nat. Rev. Genet. 2:110-119 (2001)). According to the invention, a ds RNA complex corresponding to a region of a gene to be down-regulated is expressed in the cell via a rAAV-mediated RNAi expression cassette transfer.

For the purposes of this invention, the term of "small interfering RNA" (or "siRNA") as used herein means short interfering RNA which is a double-stranded RNA complex that is less than 30 base pairs (i.e., 60 nucleotides or bases) and preferably 21-25 base pairs (i.e., 42 50 bases or nucleotides) in length. More generally, double-stranded RNA that is responsible for inducing RNAi is termed "interfering RNA". Thus, a "small interfering RNA" or "siRNA" is a double-stranded RNA complex that is capable of decreasing the expression of a gene with which it shares homology. The region of the gene or other nucleotide sequence over which there is homology is known as the "RNAi target region", "target region", "RNAi target sequence" or "target sequence". In one embodiment the siRNA may be a "hairpin" or stem-loop RNA molecule, comprising a sense region, a loop region and an antisense region complementary to the sense region and thus capable of forming an RNAi inducing dsRNA complex. In other embodiments the siRNA comprises two distinct RNA molecules that are non-covalently associated to form a dsRNA complex.

Oncogenes that may be silenced upon targeting with an RNAi expressed from a polynucleotide of interest include the oncogenes selected from the group consisting of ABLI, BCL1, BCL2, BCL6, CBFA2, CBL, CSFIR, ERBA, ERBB, EBRB2, ETS1, ETS1, ETV6, FGR, FOS, FYN, HCR, HRAS, JUN, KRAS, LCK, LYN, MDM2, MLL, MYB, MYC, MYCL1, MYCN, NRAS, PIM1, PML, RET, SRC, TAL1, TCL3 and YES.

For the purposes of this invention, the term "RNAi expression cassette" as used herein means a nucleic acid composition, which encodes one or more RNA molecules, which RNA molecules are capable of forming a double-stranded RNA complex and thus are capable of inducing RNA interference. In the context of the present invention, said RNAi expression cassette(s) are part of a rAAV vector or rAAV genome.

Thus, the present invention provides a method for decreasing or down-regulating gene expression at the mRNA level in a cancer cell of a mammalian subject in vivo. The method involves administering to a (cell of a) mammalian subject in vivo a recombinant adeno-associated viral vector with said vector comprising an RNA interference ("RNAi") expression cassette whose RNA expression product(s) directly or indirectly lead to a decrease in expression of the corresponding RNAi target gene by forming a double-stranded RNA complex which induces "RNA mediated interference" or "RNA interference" ("RNAi"), a post-transcriptional gene silencing mechanism. The dsRNA complex comprises a nucleotide sequence that hybridizes under physiologic conditions of the cell to the nucleotide sequence of at least a portion of the mRNA transcript of the gene to be down-regulated (i.e. the RNAi target gene). In particular, the RNA expression products of the RNAi expression cassette will decrease the cellular concentration of the mRNA transcript of the RNAi target gene, thus resulting in decreased concentration of the protein encoded by the RNAi target gene in the mammalian subject. Down-regulation of gene expression is specific in that a nucleotide sequence from a portion of the RNAi target gene is chosen in designing the sequence properties of the RNA coding region of the RNAi expression cassette to be transferred via rAAV-mediated gene transfer into the cells of a mammalian subject in vivo; alternatively, said: Inhibition is sequence-specific in that nucleotide sequences corresponding to the duplex region of the double-stranded RNA complex are targeted for RNA interference.

The invention also provides RNAi expression cassettes that encode the RNA molecule(s) capable of forming a double-stranded RNA complex and hence capable of inducing RNA interference in cancer cells, when operably linked to a regulatory polynucleotide derived from the human or rat AFP, human and rat HIP/PAPI that is disclosed herein. Such RNAi expression cassettes may consist of a single DNA molecule as part of a rAAV genome which, when introduced into a cell, gives rise to a single RNA molecule capable of forming intramolecularly a dsRNA complex. However, more than one rAAV genomes or RNAi expression cassettes or RNA coding regions may be introduced into a cell, either simultaneously or sequentially, to give rise to two or more RNA molecules capable of forming intermolecularly a dsRNA complex. Typically, the two RNA moieties capable of forming a dsRNA complex, whether intra- or intermolecularly, are at least in part sense and at least in part antisense sequences of a gene or nucleic acid sequence whose expression is to be down-regulated or decreased.

The design of the RNAi expression cassette does not limit the scope of the invention. Different strategies to design an RNAi expression cassette can be applied, and RNAi expression cassettes based on different designs will be able to induce RNA interference in vivo. (Although the design of the RNAi expression cassette does not limit the scope of the invention, some RNAi expression cassette designs are included in the detailed description of this invention and below.) A feature common to all RNAi expression cassettes is that they comprise an RNA coding region which encodes an RNA molecule which is capable of inducing RNA interference either alone or in combination with another RNA molecule by forming a double-stranded RNA complex either intramolecularly or intermolecularly.

Different design principles can be used to achieve that same goal and are known to those of skill in the art. For example, the RNAi expression cassette may encode one or more RNA molecules. After or during RNA expression from the RNAi expression cassette, a double-stranded RNA complex may be formed by a single, self-complementary RNA molecule or by two complementary RNA molecules. Formation of the dsRNA complex may be initiated either inside or outside the nucleus.

In one aspect there is provided a double-stranded RNA complex, which comprises, a first RNA portion capable of hybridizing to at least a portion of an mRNA molecule under physiological conditions, and a second RNA portion wherein at least a part of the second RNA portion is capable of hybridizing to the first portion under physiological conditions. Preferably the first and second portions are part of the same RNA molecule and are capable of hybridization at physiological conditions, such as those existing within a cell, and upon hybridization the first and second portions form a double-stranded RNA complex.

In another aspect there is provided a linear RNA molecule for forming a double-stranded RNA complex, which comprises a first portion capable of hybridizing to at least a portion of an mRNA molecule, preferably within a cell and a second portion wherein at least part of the second portion is capable of hybridizing to the first portion to form a hairpin dsRNA complex.

In yet another aspect, the method comprises AAV-mediated expression of RNA with partial or fully double-stranded character *in vivo.*

A dsRNA complex containing a nucleotide sequence identical to a portion of the RNAi target gene is preferred for inhibition. RNA sequences with insertions, deletions, and single point mutations relative to the RNAi target sequence have also been found to be effective for inhibition. Thus, sequence identity may be optimized by alignment algorithms known in the art and by calculating the percent difference between the nucleotide sequences. Alternatively, the duplex region of the dsRNA complex may be defined functionally as a nucleotide sequence that is capable of hybridizing with a portion of the target gene transcript.

In the preferred embodiment the RNAi expression cassette comprises at least one RNA coding region. Preferably the RNA coding region is a DNA sequence that can serve as a template for the expression of a desired RNA molecule in the host cell. In one embodiment, the RNAi expression cassette comprises two or more RNA coding regions. The RNAi expression cassette also comprises a regulatory polynucleotide derived from the human or rat AFP, or from the human or rat HIP/PAPI that is disclosed herein, so that the RNAi product is specifically expressed in mammalian cancer cells.

In certain embodiments the invention employs ribozyme-containing RNA molecules to generate dsRNA complexes, thereby overcoming certain known difficulties associated with generating dsRNA. For example, the ribozyme functionality might be used to remove polyadenylation signals, thus preventing or minimizing release of the RNA molecule from the nucleus of a cell. In other embodiments the invention is based on the ability of a portion of the RNA molecule to encode an RNA or protein that enhances specific activity of dsRNA.

In another aspect of the invention, expression of the RNA coding region results in the down regulation of a target gene. Preferably the target gene comprises a sequence that is at least about 90% identical with the RNA coding region, more preferably at least about 95% identical, and even more preferably at least about 99% identical.

The RNAi target gene does not limit the scope of this invention and may be any gene required for cell viability, including any gene required for cancerous cell viability. Thus, the choice of the RNAi target gene is not limiting for the present invention: The artisan will know how to design an RNAi expression cassette to down-regulate the gene expression of any RNAi target gene of interest. Depending on the particular RNAi target gene and the dose of rAAV virions delivered, the procedure may provide partial or complete loss of function for the RNAi target gene.

### Polynucleotides of interest encoding cytotoxic ribonucleases

For the purposes of the present invention the polynucleotide of interest that is operably linked to the regulatory region derived from the human or rat AFP, or from the human or rat HIP/PAPI promoter may consist of a polynucleotide encoding a cytotoxic ribonuclease.

Various cytotoxic ribonucleases are known in the art, including those consisting of variants of the human RNase 1, such as those having, as regards the amino acid sequence of the natural human RNase 1, one or more amino acid substitutions selected from the group consisting of N88C L86E, N88R, G89D, R91 D R4C, L86E, N88R, G89D, R91 D, V118C L86E, N88C, R91 D R4C, L86E, N88C, R91 D, V118C R4C, N88C, V118C K7A, L86E, N88C, R91 D K7A, L86E, N88R, G89D, R91 D R4C, K7A, L86E, N88C, R91 D, V118C R4C, K7A, L86E, N88R, G89D, R91 D, V118C

Cytotoxic ribonucleases encoded by a polynucleotide of interest according to the present invention also include those having, as regards the amino acid sequence of the natural human Rnase 1, one or more amino acid substitutions selected from the group consisting of :
- 7 Lysine (K), Glycine (G), Alanine (A), Aspartatic acid (D), Glutamatic acid (E), Phenylalanine (F), Tryptophan (W)
- 85 Arginine (R), Aspartatic acid (D), Glutamatic acid (E), Phenylalanine (F), Tryptophan (W), Glycine (G), Alanine (A)
- 86 Leucine (L), Aspartatic acid (D), Glutamatic acid (E), Phenylalanine (F), Lysine (K), Arginine (R), Tryptophan (W)
- 87 Threonine (T), Leucine (L), Phenylalanine (F), Tyrosine (Y), Tryptophan (W)
- 88 Asparagine (N), Lysine (K), Arginine (R), Leucine (L), Aspartic acid (D), Glutamatic acid (E), Phenylalanine (F), Tyrosine (Y), Tryptophan (W)
- 89 Glycine (G), Lysine (K), Arginine (R), Leucine (L), Aspartic acid (D), Glutamatic acid (E), Phenylalanine (F), Tyrosine (Y), Tryptophan (W)
- 90 Serine (S), Phenylalanine (F), Tryptophan (W), Aspartatic acid (D), Glutamatic acid (E), Lysine (K), Arginine (R)
- 91 Arginine (R), Aspartatic acid (D), Glutamatic acid (E), Phenylalanine (F), Tryptophan (W)
- 92 Tyrosine (Y), Glycine (G), Alanine (A), Lysine (K), Arginine (R), Aspartic acid (D), Glutamatic acid (E)
- 93 Proline (P), Leucine (L), Phenylalanine (F), Tyrosine (Y), Tryptophan (W), Lysine (K), Arginine (R), Aspartic acid (D), Glutamatic acid (E)
- 94 Asparagine (N), Lysine (K), Arginine (R), Leucine (L), Aspartic acid (D), Glutamatic acid (E), Phenylalanine (F), Tyrosine (Y), Tryptophan (W)

Cytotoxic ribonucleases encoded by a polynucleotide of interest according to the present invention also include those having, as regards the amino acid sequence of the natural human RNase 1, one or more amino acid substitutions selected from the group consisting of :
- 1 Lysine (K), Cysteine (C)
- 2 Glutamic acid (E), Cysteine (C)
- 3 Serine (S), Cysteine (C)
- 4 Arginine (R), Cysteine (C)
- 5 Alanine (A), Cysteine (C)
- 6 Lysine (K), Cysteine (C)
- 116 Valine (V), Cysteine (C)
- 117 Proline (P), Cysteine (C)
- 118 Valine (V), Cysteine (C)
- 119 Histidine (H), Cysteine (C)
- 120 Phenylalanine (F), Cysteine (C), and
- 121 Aspartate (D), Cysteine (C)

Various cytotoxic ribonucleases, including those listed above, are described in the US patent application n° US 2005 261 232, which is herein incorporated in its entirety by reference.

### Polynucleotides of interest encoding ribozymes

Ribozymes, as well as their construction and use, are known in the art. See, e.g. Norris et al., 2000, "Design and testing of ribozymes for cancer gene therapy," Adv. Exp Med. Biol. 465:293-301. See also, Cech, U.S. Pat. No. 5,093,246; Cech, U.S. Pat. No. 5,116,742; Haselhoff et al., 1988, Nature 334:585-591. Employing known nucleotide sequences of metallothionein mRNA molecules together with known properties of ribozymes in general, numerous different ribozymes or ribozyme-encoding vectors useful in the invention can be designed and produced. Ribozymes that may be encoded by a polynucleotide of interest according to the invention include those interfering with metallothionein gene expression, such as disclosed in the US patent application n° US 2003 105 043, which is herein incorporated in its entirety by reference. Such ribozymes induce apoptosis in targeted cancer cells. In addition, the ribozymes render targeted cancer cells more susceptible to radiation, reactive oxygen species and chemotherapeutic agents. Thus, specific expression of such ribozyme in mammalian cancer cells can be employed alone or in combination with conventional radiation therapy or chemotherapy. Ribozymes targeted to mRNAs representing one or more of the 17 human metallothionein genes can be used to treat various human cancers, including prostate cancer, breast cancer, and ovarian cancer.

Such ribozymes destroy metallothionein-encoding mRNAs, rather than merely hybridizing with them. Ribozymes act like enzymes and each molecule can be "recycled" to degrade multiple mRNA molecules. A further advantage is that a ribozyme need not have perfect complementarity with a target mRNA in order to destroy the RNA. Hybridization need only be sufficient to enable the catalytic site of the ribozyme to cleave the target mRNA. Therefore, a single ribozyme can be designed to destroy several related mRNAs that encode different metallothioneins more readily than a conventional antisense molecule can be designed to be effective against various mRNAs.

Also encompassed herein are those polynucleotides of interest that encode for ribozymes that specifically cleave matrix metalloproteinase 13 (MMP-13) mRNA, such as disclosed in the US patent application n° 2003 195 164, which is herein incorporated in its entirety by reference.

### Polynucleotides of interest encoding sense or antisense nucleic acids.

Polynucleotides of interest according to the invention may also encode for sense or antisense polynucleotides that interfere with expression of specific target genes in view of inducing an anti-cancer activity.

Such polynucleotides of interest include those encoding antisense polynucleotides that are disclosed in the US patent applications n° 2005 261 249 or n° 2005 113 328, which are incorporated herein in their entirety by reference.

Such polynucleotides of interest also include those encoding antisense polynucleotides that are complementary to nucleolin mRNA and that are disclosed in the US patent application n° 2005 26 860, which is incorporated herein in its entirety by reference.

Such polynucleotides of interest also include those encoding antisense polynucleotides that are complementary to BRCA-1 mRNA and that are disclosed in the US patent application n° 2005 26 857, which is incorporated herein in its entirety by reference.

Such polynucleotides of interest also include those encoding antisense polynucleotides that are complementary to WT-1 mRNA and that are disclosed in the US patent application n° 2004 43 950, which is incorporated herein in its entirety by reference.

Such polynucleotides of interest also include those encoding antisense polynucleotides that are complementary to kinesin mRNA and that are disclosed in the US patent application n° 2004 9 156, which is incorporated herein in its entirety by reference.

Such polynucleotides of interest also include those encoding antisense polynucleotides that are complementary to testosterone-repressed message-2 (TRPM-2) mRNA and that are disclosed in the US patent application n° 2003 158 130, which is incorporated herein in its entirety by reference.

Such polynucleotides of interest also include those encoding antisense polynucleotides that consists of ADNF-III oligonucleotides and that are disclosed in the US patent application n° 2003 36 521, which is incorporated herein in its entirety by reference.

### Most preferred embodiments of a polynucleotide of interest according to the invention

In a most preferred embodiment, the polynucleotide of interest that is operably linked to a regulatory sequence derived from the human AFP promoter, from the rat AFP promoter, from the human HIP/PAPI promoter or from the rat HIP/PAPI promoter consists of a polynucleotide encoding the rat sodium iodide symporter (NIS) protein which catalyses iodine cell uptake.

It has already been shown in the art that, in rat cells that over-express the NIS gene, high levels of radio-iodine cell uptake were reached. When recombinant vectors expressing NIS were injected directly into cancer nodules of hepatocarcinoma-bearing rats, an inhibition of the tumour growth was measured (2004, Faivre et al., Cancer Research, Vol. 64 : 8045-8051). However, the recombinant vectors designed by Faivre et al. (2004) did not allow expression of the NIS protein specifically in cancerous cells and thus were not usable for therapy.

In contrast, when using a nucleic acid according to the invention comprising, as the polynucleotide of interest, a polynucleotide encoding NIS, the NIS protein is expressed specifically in the targeted cancerous cells.

Further, using a nucleic acid comprising a NIS-encoding polynucleotide of interest that is operably linked to one regulatory polynucleotide that is defined in the present specification, the high level of radioiodine cell uptake is sufficient *per se* for specifically killing tumour cells and inhibiting tumour growth, without any need to simultaneously express one or more genes encoding proteins that retain the cytotoxic radionuclide(s) at the cell level, like, for example, the gene encoding thyroid peroxidase (TPO) or the gene encoding thyroglobulin (TG).

In certain embodiments of a polynucleotide of interest, the said polynucleotide of interest encodes the NIS protein disclosed by Faivre et al. (2004, Faivre et al., Cancer Research, Vol. 64 : 8045-8051).

In certain embodiments of a polynucleotide of interest, the said polynucleotide of interest encodes the NIS protein disclosed in the PCT application n° WO 97/28175.

### EXPRESSION CASSETTES

As used herein, "expression cassettes" consists of nucleic acids comprising at least the following two polynucleotides, respectively : (i) a first polynucleotide consisting of a regulatory polynucleotide derived from the human or rat AFP promoter, or from the human or rat HIP/PAPI promoter as defined herein and (ii) a second polynucleotide consisting of a polynucleotide of interest, as defined herein, that induces, directly or indirectly, anti-cancer activity.

Consequently, in view of the general definition of an expression cassette given above, a "nucleic acid" according to the invention also consists of one specific embodiment of an expression cassette.

An expression cassette of the present invention may also contain a polyadenylation signal operably linked to the polynucleotide of interest. A polyadenylation sequence is operably linked to the polynucleotide to be transcribed when it allows termination of transcription. It is preferably positioned 3' (downstream) of the polynucleotide of interest. A suitable polyadenylation signal includes the beta growth hormone (bGHpA), the SV40 and the beta-globin polyadenylation signals.

One preferred expression cassette according to the invention comprises, from 5'-end to 3'-end, respectively (i) one regulatory polynucleotide as defined herein and (ii) one polynucleotide of interest encoding the NIS protein, respectively.

The most preferred expression cassette according to the invention comprises, from 5'-end to 3'-end, (i) one regulatory polynucleotide derived from the human HIP/PAPI promoter that is described herein and (ii) one polynucleotide of interest encoding the NIS protein.

The expression cassette may further contain a marker gene. The expression cassette may be contained in a vector.

### RECOMBINANT VECTORS

The invention provides vectors and methods for vector-mediated delivery and expression of anti-cancer factors that are effective in the treatment of cancer. In particular, the invention relates to the use of recombinant viral and non-viral vectors to deliver a nucleic acid for expressing a polynucleotide of interest specifically in cancer cells, to a subject for the treatment of cancer.

Thus, another object of the present invention consists of recombinant vectors having inserted therein a nucleic acid for expressing a polynucleotide of interest specifically in mammalian cancer cells.

For purposes of this invention, by "vector", "transfer vector", "gene transfer vector" or "nucleic acid composition transfer vector" is meant any element, such as a plasmid, phage, transposon, cosmid, chromosome, virus, virion, etc., which is capable of transferring and/or transporting a nucleic acid composition to a host cell, into a host cell and/or to a specific location and/or compartment within a host cell. Thus, the term includes cloning and expression vehicles, as well as viral and non-viral vectors and potentially naked or complexed DNA. However, the term does not include cells that produce gene transfer vectors such as retroviral packaging cell lines.

For mammalian host cells, viral-based and non-viral expression systems are provided. Non-viral vectors and systems include plasmids and episomal vectors, typically with an expression cassette comprising a nucleic acid according to the invention, and human artificial chromosomes (see, e.g., Harrington et al., 1997, Nat Genet 15:345). For example, non-viral vectors useful for expression of a nucleic acid according to the invention in mammalian (e.g., human) cells include pcDNA3.1/His, pEBVHis A, B & C, (Invitrogen, San Diego Calif.), MPSV vectors, others described in the Invitrogen 1997 Catalog (Invitrogen Inc, San Diego Calif.), which is incorporated in its entirety herein, and numerous others known in the art for other proteins.

### Viral vectors

Useful viral vectors include vectors based on retroviruses, adenoviruses, adeno-associated viruses, herpes viruses, vectors based on SV40, papilloma virus, HBP Epstein Barr virus, vaccinia virus vectors and Semliki Forest virus (SFV). SFV and vaccinia vectors are discussed generally in Ausubel et al., supra, Ch 16. These vectors are often made up of two components, namely a modified viral genome and a coat structure surrounding it (see generally Smith, 1995, Annu. Rev. Microbiol. 49: 807), although viral vectors are sometimes introduced in naked form or coated with proteins other than viral proteins. However, the viral nucleic acid in a vector may be changed in many ways, for example, when designed for gene therapy. The goals of these changes are to disable growth of the virus in target cells while maintaining its ability to grow in vector form in available packaging or helper cells, to provide space within the viral genome for insertion of exogenous DNA sequences, and to incorporate new sequences that encode and enable appropriate expression of the gene of interest. Thus, vector nucleic acids generally comprise two components, namely essential cis-acting viral sequences for replication and packaging in a helper line and the transcription unit for the exogenous gene. Other viral functions are expressed in trans in a specific packaging or helper cell line. Adenoviral vectors (e.g., for use in human gene therapy) are described in, e.g., Rosenfeld et al., 1992, Cell 68: 143; PCT publications WO 94/12650; 94/12649; and 94/12629. In cases when an adenovirus is used as an expression vector, a nucleic acid according to the invention may be cloned into an adenovirus transcription/translation complex consisting of the late promoter and tripartite leader sequence. Insertion in a nonessential E1 or E3 region of the viral genome will result in a viable virus capable of expressing a therapeutic polynucleotide sequence in infected host cells (Logan and Shenk, 1984, Proc. Natl. Acad. Sci., 81:3655). Replication-defective retroviral vectors harboring a therapeutic polynucleotide sequence as part of the retroviral genome are described in, e.g., Miller et al., 1990, Mol. Cell. Biol. 10: 4239; Kolberg, 1992, J. NIH Res. 4: 43; and Cornetta et al., 1991, Hum. Gene Ther. 2: 215.

### Adenoviral vectors

It is preferable to use an adenovirus vector as a gene carrier in the present invention.

Adenovirus is a DNA virus, whose genomic DNA is approximately 36 kbp in size. Adenovirus does not harm humans but the E1 gene of adenoviral genome has transformation potential in rodent. Therefore, this region must be removed to enhance safety of the treatment.

To construct recombinant adenovirus as a gene delivery vector, E1A and E1B region are replaced with a gene of interest that makes of it a replication-defective adenovirus called a first-generation adenovirus vector. E3 region can be additionally eliminated when the insert gene is longer than 3.5 kbp. Recombinant adenovirus without E1 region can be propagated in 293 cells expressing E1A and E1 B proteins constitutively.

To construct a recombinant adenovirus that can express a polynucleotide of interest as defined above specifically in cancer cells, one may eliminate E1 region from the adenovirus genomic DNA and insert instead an expression cassette including the nucleic acid of the invention.

### Adeno-associated virus vectors

In one embodiment, the recombinant vector consists of an AAV vector comprising a nucleic acid for expressing a polynucleotide of interest specifically in cancer cells, as defined in the present specification.

For the purposes of this invention, by "AAV vector", "AAV-based vector", "adeno-associated virus based vector", "adeno-associated viral vector", "rAAV vector" or "recombinant adeno-associated viral vector" is meant a vector derived from an adeno-associated virus serotype, including without limitation, AAV-1, AAV-2, AAV-3, AAV-4, AAV-5, AAV-6, AAV-7, AAV-8 etc. or any other virus or serotype that is substantially homologous in its capsid protein sequence to the AAV2 or AAV5 capsid protein sequence. The term also includes hybrid vectors combining characteristics of more than one AAV serotype. AAV vectors can have one or more of the AAV wild-type genes deleted in whole or part, preferably the rep and/or cap genes, but retain functional flanking ITR sequences. Functional ITR sequences are necessary for the rescue, replication and packaging of the AAV virion. Thus, an AAV vector is defined herein to include at least those sequences required in cis for replication and packaging (e.g. functional ITRs) of the virus. The ITRs need not be the wild-type nucleotide sequences, and may be altered, e.g. by the insertion, deletion or substitution of nucleotides, as long as the sequences provide for functional rescue, replication and packaging.

For the purposes of this invention, by "AAV vector construct" or "rAAV vector construct" is meant a nucleic acid composition that is used in the production of rAAV virions (rAAV vectors). More specifically, rAAV vector constructs give rise to the rAAV genomes to be packaged into the rAAV capsids. AAV vector constructs are constructed using known techniques to provide, as operatively linked components in the direction of transcription, (a) a regulatory polynucleotide derived from the human or rat AFP promoter, or from the human or rat HIP/PAPI promoter, (b) the polynucleotide of interest (e.g. a sequence encoding a cytotoxic protein, including NIS protein or a toxic ribonuclease, a sequence encoding a ribozyme, an antisense polynucleotide, etc.), and (c) a transcriptional termination region. The resulting construct, which contains the operatively linked components, is bounded (5' and 3') with functional AAV ITR sequences. As an example, an AAV vector construct can be a double-stranded DNA plasmid.

For the purposes of this invention, by "recombinant virus", "recombinant virion", "recombinant vector" or "recombinant viral vector" is meant a virus that has been genetically altered, e.g. by the addition or insertion of a heterologous nucleic acid composition into the particle.

For the purposes of this invention, by "AAV virion" is meant a complete virus particle, such as a wild-type (wt) AAV virus particle (comprising a linear, single-stranded AAV nucleic acid genome associated with an AAV capsid protein coat). In this regard, single-stranded AAV nucleic acid molecules of either complementary sense, e.g. "sense" or "antisense" strands, can be packaged into any one AAV virion, and both strands are equally infectious.

For the purposes of this invention, a "recombinant AAV virion" or "rAAV virion" is defined herein as an infectious, replication-defective virus composed of an AAV protein shell, encapsidating a heterologous DNA molecule of interest, which is flanked on one or both sides by AAV ITRs. A rAAV virion is produced in a suitable host cell, which has had an AAV vector construct, AAV helper functions and accessory functions introduced therein. In this manner, the host cell is rendered capable of encoding the AAV polypeptides that are required for packaging the AAV vector (comprising a recombinant nucleotide sequence of interest) into recombinant virion particles for subsequent gene delivery. The term "rAAV virion" and its synonyms and the term "rAAV vector" and its synonyms can be used interchangeably unless the context clearly dictates otherwise.

For the purposes of this invention, "pseudotyped" (r)AAV refers to a recombinant AAV in which the capsid protein is of a serotype heterologous to the serotype(s) of the ITRs of the minigene. For example, a pseudotyped rAAV may be composed of a minigene carrying AAV5 ITRs and cap sid of AAV2, AAV1, AAV3, AAV4, AAV6, AAV7, AAV8 or another suitable AAV serotype, where the minigene is packaged in the heterologous capsid. Alternatively, a pseudotyped rAAV may be composed of an AAV5 capsid, which has packaged therein a minigene containing ITRs from at least one of the other serotypes. Particularly indicated rAAV composed of AAV5 are described in U.S. patent application Ser. No. 60/200,409, filed Apr. 28, 2000 and International Patent Application No. PCT/USO1/13000, filed Apr. 23, 2001, both of which are incorporated by reference herein.

### Most preferred recombinant vectors

Most preferred vectors according to this invention consist of adenoviral vectors containing, inserted in their DNA material, at least one nucleic acid for expressing a polynucleotide of interest specifically in cancer cells as defined herein, or at least one expression cassette as defined herein.

In certain embodiments, the adenoviral vectors according to the invention may be prepared according to the technique disclosed by Tong-Chuan et al. (1998, Proc. Natl. Acad. Sci. USA, Vol. 95 : 2590-2514), the same technique being also disclosed in the US patent n° US 5,922,576 which is herein incorporated by reference in its entirety.

In certain embodiments, the adenoviral vectors according to the invention may be prepared according to the general technique disclosed by Faivre et al. (2004, Cancer Research, Vol. 64 : 8045-8051), using a nucleic acid or an expression cassette as defined herein as the DNA insert.

### RECOMBINANT HOST CELLS

Any one of the recombinant vectors described above, that comprises a nucleic acid according to the invention for expressing a polynucleotide of interest specifically in cancer cells, is useful for *in vivo* administration to mammals, specifically to humans, in need thereof. In some instances, these vectors may also be used *in vitro* for transfecting cancer cells, e.g. for ensuring, by *in vitro* assays, the anti-cancer efficiency of a determined polynucleotide of interest, before clinical use.

Thus, another object of the present invention consists of a recombinant host cell that has been transfected by a nucleic acid according to the invention, including by cell transfection with a recombinant vector such as defined above.

For the purposes of this invention, "transfection" is used to refer to the uptake of nucleic acid compositions by a cell. A cell has been "transfected" when an exogenous nucleic acid composition has crossed the cell membrane. A number of transfection techniques are generally known in the art. See, e.g. Sambrook et al. (1989) Molecular Cloning, a laboratory manual, Cold Spring Harbor Laboratories, New York, Davis et al. (1986) Basic Methods in Molecular Biology, Elsevier. Such techniques can be used to introduce one or more nucleic acid compositions, such as a plasmid vector and other nucleic acid molecules, into suitable host cells. The term refers to both stable and transient uptake of the genetic material. For purposes of this invention, "transduction" is a special form of "transfection" via a viral vector.

For the purposes of this invention, "transduction" denotes the delivery of a nucleic acid composition to, into or within a recipient cell either in vivo, in vitro or ex vivo, via a virus or viral vector, such as a recombinant AAV virion. Transduction is a special form of transfection, i.e., the term transfection includes the term transduction.

Thus, generally, recombinant host cells according to the invention consist of a cancerous cells that intra-cellularly bear a recombinant vector as defined above and express the polynucleotide of interest, the expression of which is under the control of a regulatory polynucleotide derived from the human or rat AFP promoter, or from the human or rat HIP/PAPI promoter.

### PHARMACEUTICAL COMPOSITIONS AND THERAPEUTIC METHODS

Another object of the present invention consists of pharmaceutical compositions comprising at least an active ingredient selected from the group consisting of (i) a nucleic acid for expressing a polynucleotide of interest specifically in cancer cells as defined herein, (ii) an expression cassette comprising the said nucleic acid according to the invention, and (iii) a recombinant vector in which is inserted the said nucleic acid or the said expression cassette.

This invention also relates to anti-cancer therapeutic methods comprising a step of administering to a mammal body, specifically to a human body, in need thereof, a pharmaceutical composition such as generally defined above.

Thus, another object of the present invention consists of a pharmaceutical composition comprising :
(i) an active ingredient selected from the group consisting of :
   - a nucleic acid or an expression cassette such as described herein; and
   - a recombinant vector such as defined herein;
      and
(ii) optionally, one or more pharmaceutically compatible excipients.

This invention also relates to the use of a nucleic acid or an expression cassette such as described herein for manufacturing a pharmaceutical composition.

This invention also pertains to the use of a recombinant vector such as defined herein for manufacturing a pharmaceutical composition.

Methods for the delivery of nucleic acid molecules are described in Akhtar et al., 1992, Trends Cell Bio., 2, 139; and Delivery Strategies for Antisense Oligonucleotide Therapeutics, ed. Akhtar, 1995 which are both incorporated herein by reference. Sullivan et al., PCT WO 94/02595, further describes the general methods for delivery of enzymatic RNA molecules. These protocols can be utilized for the delivery of virtually any nucleic acid molecule. Nucleic acid molecules can be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. Alternatively, the nucleic acid/vehicle combination is locally delivered by direct injection or by use of an infusion pump. Other routes of delivery include, but are not limited to, oral (tablet or pill form) and/or intrathecal delivery (Gold, 1997, Neuroscience, 76, 1153-1158). Other approaches include the use of various transport and carrier systems, for example through the use of conjugates and biodegradable polymers. For a comprehensive review on drug delivery strategies including CNS delivery, see Ho et al., 1999, Curr. Opin. Mol. Ther., 1, 336-343 and Jain, Drug Delivery Systems: Technologies and Commercial Opportunities, Decision Resources, 1998 and Groothuis et al., 1997, J NeuroVirol., 3, 387-400. More detailed descriptions of nucleic acid delivery and administration are provided in Sullivan et al., supra, Draper et al., PCT WO93/23569, Beigelman et al., PCT WO99/05094, and Klimuk et al., PCT WO99/04819, all of which have been incorporated by reference herein.

The nucleic acids, expression cassettes and recombinant vectors of the present invention can be used as pharmaceutical agents.

The nucleic acids, expression cassettes and recombinant vectors of the invention can be administered and introduced into a patient by any standard means, with or without stabilizers, buffers, and the like, to form a pharmaceutical composition. When it is desired to use a liposome delivery mechanism, standard protocols for formation of liposomes can be followed. The compositions of the present invention can also be formulated and used as sterile solutions; suspensions for injectable administration; and the other compositions known in the art.

The present invention also includes pharmaceutically acceptable formulations of the active substances described.

A pharmacological composition or formulation refers to a composition or formulation in a form suitable for administration, e.g., systemic administration, into a cell or a patient, preferably a human. Suitable forms, in part, depend upon the use or the route of entry. For example, pharmacological compositions injected into the blood stream should be soluble. Other factors are known in the art, and include considerations such as toxicity and forms, which prevent the composition or formulation from exerting its effect.

By "systemic administration" is meant in vivo systemic absorption or accumulation of drugs in the blood stream followed by distribution throughout the entire body. Administration routes, which lead to systemic absorption include, without limitations: intravenous, subcutaneous, intraperitoneal, inhalation, oral, intrapulmonary and intramuscular. Each of these administration routes exposes the desired negatively charged polymers, e.g., nucleic acids, to an accessible diseased tissue. The rate of entry of a drug into the circulation has been shown to be a function of molecular weight or size. The use of a liposome or other drug carrier comprising the compounds of the instant invention can potentially localize the drug, for example, in certain tissue types, such as the tissues of the reticular endothelial system (RES). A liposome formulation, which can facilitate the association of drug with the surface of cells, including cancer cells is also useful.

By pharmaceutically acceptable formulation is meant, a composition or formulation that allows for the effective distribution of the nucleic acid molecules or the recombinant vectors of the present invention in the physical location the most suitable for their desired activity. Non-limiting examples of agents suitable for formulation with the nucleic acid molecules of the present invention include: PEG conjugated nucleic acids, phospholipid conjugated nucleic acids, nucleic acids containing lipophilic moieties, phosphorothioates, P-glycoprotein inhibitors (such as Pluronic P85), which can enhance entry of drugs into various tissues, for example the CNS (Jolliet-Riant and Tillement, 1999, Fundam. Clin. Pharmacol., 13, 16-26); biodegradable polymers, such as poly (DL-lactide-coglycolide) microspheres for sustained release delivery after implantation (Emerich, DF et al, 1999, Cell Transplant, 8, 47-58) Alkermes, Inc. Cambridge, Mass.; and loaded nanoparticles, such as those made of polybutylcyanoacrylate, which can deliver drugs across the blood brain barrier and can alter neuronal uptake mechanisms (Prog Neuropsychopharmacol Biol Psychiatry, 23, 941-949, 1999). Other non-limiting examples of delivery strategies, including CNS delivery of the nucleic acid molecules of the instant invention include material described in Boado et al., 1998, J. Pharm. Sci., 87, 1308-1315; Tyler et al., 1999, FEBS Lett., 421, 280-284; Pardridge et al., 1995, PNAS USA., 92, 5592-5596; Boado, 1995, Adv. Drug Delivery Rev., 15, 73-107; Aldrian-Herrada et al., 1998, Nucleic Acids Res., 26, 4910-4916; and Tyler et al., 1999, PNAS USA., 96, 7053-7058. All these references are hereby incorporated herein by reference.

The present invention also features the use of the composition comprising surface-modified liposomes containing poly (ethylene glycol) lipids (PEG-modified, or long-circulating liposomes or stealth liposomes). Nucleic acid molecules of the invention can also comprise covalently attached PEG molecules of various molecular weights. These formulations offer a method of increasing the accumulation of drugs in target tissues. This class of drug carriers resists opsonization and elimination by the mononuclear phagocyte system (MPS or RES), thereby enabling longer blood circulation times and enhanced tissue exposure for the encapsulated drug (Lasic et al. Chem. Rev. 1995, 95, 2601-2627; Ishiwata et al., Chem. Pharm. Bull. 1995, 43, 1005-1011). Such liposomes have been shown to accumulate selectively in tumours, presumably by extravasation and capture in the neovascularized target tissues (Lasic et al., Science 1995, 267, 1275-1276; Oku et al., 1995, Biochim. Biophys. Acta, 1238, 86-90). The long-circulating liposomes enhance the pharmacokinetics and pharmacodynamics of DNA and RNA, particularly compared to conventional cationic liposomes, which are known to accumulate in tissues of the MPS (Liu et al., J. Biol. Chem. 1995, 42, 24864-24870; Choi et al., International PCT Publication No. WO 96/10391; Ansell et al., International PCT Publication No. WO 96/10390; Holland et al., International PCT Publication No. WO 96/10392; all of which are incorporated by reference herein). Long-circulating liposomes are also likely to protect drugs from nuclease degradation to a greater extent than cationic liposomes, based on the ability of the former to avoid accumulation in metabolically aggressive MPS tissues such as the liver and spleen. All of these references are incorporated by reference herein.

The present invention also includes compositions prepared for storage or administration, which include a pharmaceutically effective amount of the desired compounds in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (A. R. Gennaro edit. 1985) hereby incorporated by reference herein. For example, preservatives, stabilizers, dyes and flavouring agents can be provided. These include sodium benzoate, sorbic acid and esters of p-hydroxybenzoic acid. In addition, antioxidants and suspending agents can be used.

A pharmaceutically effective dose is the dose that is required to prevent, inhibit the occurrence, or treat the corresponding patient's cancer state. The pharmaceutically effective dose depends on the type of cancer, the cancer development status, the composition used, the route of administration, the type of mammal being treated, the physical characteristics of the specific mammal under consideration, concurrent medication, and other factors, which those skilled in the medical arts will recognize. Generally, an amount of a nucleic acid, of an expression cassette or of a recombinant vector between 0.1 µg/kg and 10 mg/kg body weight/day of active ingredients is administered dependent upon potency of the negatively charged polymer.

The nucleic acid molecules and recombinant vectors of the invention and formulations thereof can be administered parenterally, in dosage unit formulations containing conventional non-toxic pharmaceutically acceptable carriers, adjuvants and vehicles. The term parenteral as used herein includes percutaneous, subcutaneous, intravascular (e.g. intravenous), intramuscular, or intrathecal injection or infusion techniques and the like.

In a pharmaceutical composition of the invention, a number of nucleic acid molecules or recombinant vectors of the invention can be present in combination with one or more non-toxic pharmaceutically acceptable carriers and/or diluents and/or adjuvants, and if desired other active ingredients.

Aqueous suspensions contain the active materials in admixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Nucleic acid molecules and recombinant vectors of the invention can be administered parenterally in a sterile medium. The drug, depending on the vehicle and concentration used, can either be suspended or dissolved in the vehicle. Advantageously, adjuvants such as local anaesthetics, preservatives and buffering agents can be dissolved in the vehicle.

It is understood that the specific dose level for any particular patient depends upon a variety of factors including the activity of the specific compound employed, the age, body weight, general health, sex, diet, time of administration, route of administration, and rate of excretion, drug combination and the severity of the particular cancer undergoing therapy.

The nucleic acid molecules and the recombinant vectors of the present invention can also be administered to a patient in combination with other therapeutic compounds to increase the overall anti-cancer therapeutic effect.

The pharmaceutical compositions of the present invention can be used for therapeutic treatment for cancers of any type, including solid tumours and leukemias, including: apudoma, choristoma, branchioma, malignant carcinoid syndrome, carcinoid heart disease, carcinoma (e.g., Walker, basal cell, basosquamous, Brown-Pearce, ductal, Ehrlich tumour, in situ, Krebs 2, Merkel cell, mucinous, non-small cell lung, coat cell, papillary, scirrhous, bronchiolar, bronchogenic, squamous cell, and transitional cell), histiocytic disorders, leukaemia (e.g. B cell, mixed cell, null cell, T cell, T-cell chronic, HTLV-II-associated, lymphocytic acute, lymphocytic chronic, mast cell, and myeloid), histiocytosis malignant, Hodgkin disease, immunoproliferative small, non-Hodgkin lymphoma, plasmacytoma, reticuloendotheliosis, melanoma, chondroblastoma, chondroma, chondrosarcoma, fibroma, fibrosarcoma, giant cell tumours, histiocytoma, lipoma, liposarcoma, mesothelioma, myxoma, myxosarcoma, osteoma, osteosarcoma, Ewing sarcoma, synovioma, adenofibroma, adenolymphoma, carcinosarcoma, chondroma, cranio-pharyngioma, dysgerminoma, hamartoma, mesenchymoma, mesonephroma, myosarcoma, ameloblastoma, cementoma, odontoma, teratoma, thymoma, trophoblastic tumour, adenocarcinoma, carcinoma, adenoma, cholangioma, cholesteatoma, cylindroma, cystadenocarcinoma, cystadenoma, granulosa cell tumour, gynandroblastoma, hepatoma, hidradenoma, islet cell tumour, Leydig cell tumour, papilloma, Sertoli cell tumour, theca cell tumour, leiomyoma, leiomyosarcoma, myoblastoma, myoma, myosarcoma, rhabdomyoma, rhabdomyosarcoma, ependymoma, ganglioneuroma, glioma, medulloblastoma, meningioma, neurilemmoma, neuroblastoma, neuroepithelioma, neurofibroma, neuroma, paraganglioma, nonchromaffin, angiokeratoma, angiolymphoid hyperplasia with eosinophilia, angioma sclerosing, angiomatosis, glomangioma, hemangioendothelioma, hemangioma, hemangiopericytoma, hemangiosarcoma, lymphangioma, lymphangiomyoma, lymphangiosarcoma, pinealoma, carcinosarcoma, chondrosarcoma, cystosarcoma phyl lodes, fibrosarcoma, hemangiosarcoma, leiomyosarcoma, leukosarcoma, liposarcoma, lymphangiosarcoma, myosarcoma, myxosarcoma, ovarian carcinoma, rhabdomyosarcoma, sarcoma (e.g. Ewing, experimental, Kaposi, and mast cell), neoplasms (e.g. bone, breast, digestive system, colorectal, liver, pancreatic, pituitary, testicular, orbital, head and neck, central nervous system, acoustic, pelvic, respiratory tract, and urogenital), neurofibromatosis, and cervical dysplasia, and for treatment of other conditions in which cells have become immortalized or transformed. The invention could be used in combination with other treatment modalities, such as chemotherapy, cryotherapy, hyperthernia, radiation therapy, and the like.

The present invention is further illustrated by the examples below.

### EXAMPLES

### Example 1 : Preparation of the nucleic acids comprising a regulatory polynucleotide derived from the human HIP/PAPI and the rat PAPI promoters.

Human and rat HIP/PAPI (Hepatocarcinoma-Intestine-Pancreas/Pancreatitis Associated Protein I) promoter mutants were generated as follows.

A fragment of 2.44 kb encompassing the full length regulatory sequence of human HIP/PAPI gene comprising exon 1 was released from pCL6 vector by EcoRI-BamHI digestion.

Various mutants of deletion were blunt-ended with Klenow polymerase, kinased, and ligated into the *Sal*I site of the pLuciferase292 vector (pL292, Promega) to generate the plasmids pL292-EcoRI-HIP/PAPI, pL292-XmnI-HIP/PAPI, pL292-PstI-HIP/PAPI, pL292-Stul-HIP/PAPI and pL292-Bsu36I-HIP/PAPI. These luciferase reporter gene constructs contained nucleotides from position 2092 to +348, 1714 to +348, 987 to +348, 238 to +348 and +106 to +348 of the human HIP/PAP I promoter region, respectively (the transcription initiation site is numbered +1). A Luciferase assay was then performed to determine the mutant of deletion harbouring the highest transcriptional activity.

A fragment of 1.253 Kb of the rat HIP/PAPI promoter, and a fragment of 2 Kb of the selected human HIP/PAPI promoter were amplified by polymerase chain reaction using the plasmids p-1253/+10PAPI-CAT (p-1253/+10PAPI-CAT vector was formerly disclosed by Dusetti et al., 1995, J Biol Chem, Vol. 270(38) : 22417-22421) and pL292-Xmnl-HIP/PAPI as templates, respectively.

Amplification was performed in 1X polymerase chain reaction buffer containing 0.2 mM dNTP, 0.2 µmol of each primer and 2.5 units of Expand high fidelity Taq polymerase (Roche) in a final volume of 50 µL. PCR reaction was performed during 35 cycles at 94°C for 60s, 55°C for 30s and 72°C for 120s.

The following oligonucleotides were used:

### Human HIP/PAPI :

- sense primer carrying a KpnI restriction site 5'-CGGGGTACCACTGCAGCCTTGAACTCCTGG-3' [SEQ ID N° 17]',
- antisense primer carrying a SalI restriction site 5'-ACGGTCGACTGTCTGCGACTTGAGGAGGCA-3' [SEQ ID N° 18];

### Rat HIP/PAP :

- sense primer carrying a SalI restriction site 5'-CCGCGTCGAGCTGCAGATTTTCCAGTTAGTC-3' [SEQ ID N° 19],
- antisense primer carrying a Mlul restriction site 5'-TCGACGCGTGTGAGGACAGAGATGGCTGTG-3' [SEQ ID N° 20].

The pSVSport-rNIS and the pShuttle were kindly provided by Pr. N. Carrasco (Albert Einstein College of Medicine, Bronx, New York) and the vector core of the University Hospital of Nantes, respectively.

The rat NIS cDNA was excised from pSVSport-rNIS using SalI-EcoRV (New England Biolabs) blunt ended digestion, purified on agarose gel and ligated (T₄ DNA ligase, Roche) into the SalI-BgIII blunt ended pShuttle resulting in the pShuttle-rNIS. The human and the rat HIP/PAPI PCR products were inserted into the pShuttle-rNIS.

The pShuttle-rNIS and hHIP PCR products were digested by KpnI (10 units of enzyme per 10µg of DNA) during 2 hours at 37°C, precipitated overnight at -20°C in absolute ethanol and sodium acetate 3M, and digested by SalI during 2 hours at 37°C. After migration and purification on agarose gel (QiaExII, Qiagen), the DNAs were ligated at 16°C overnight using T₄ DNA ligase to generate the pShuttle hHIP-rNIS.

The same experimental procedure was used to generate the pShuttle rHIP-rNIS after digestion of pShuttle-rNIS and rHIP PCR products by SalI-MluI followed by purification and ligation.

### Example 2 : Preparation of the nucleic acids comprising a regulatory polynucleotide derived from the human AFP promoter.

The pShuttle rAFPwt- and hAFPt-rNIS were constructed by using convenient restriction enzymes.

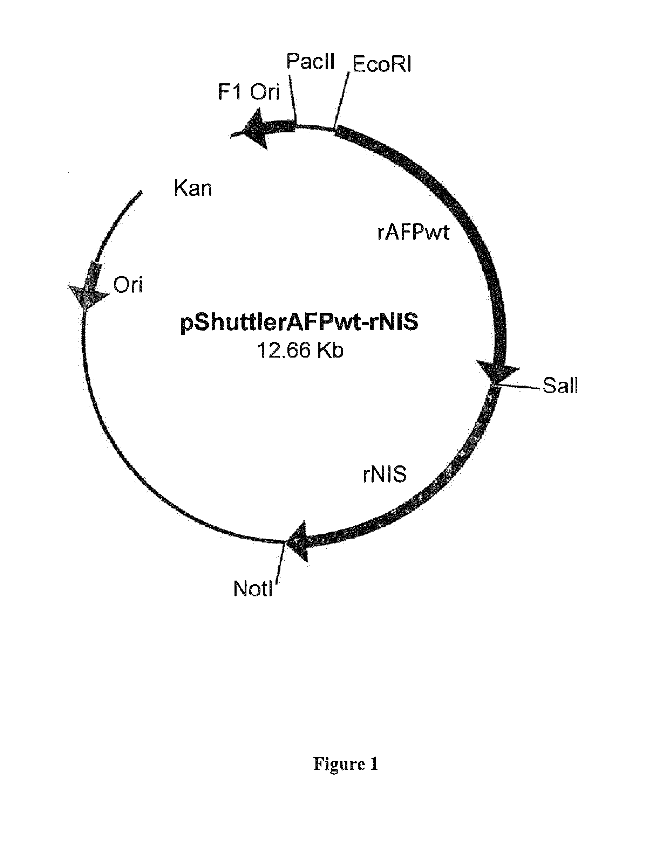

To generate pShuttle rAFPwt-rNIS (Figure 1), the EcoRI-SalI 3.2 Kb fragment with the rat alpha-fetoprotein (rAFP) transcriptional regulatory sequence was released from pBluescript-rAFPwt, inserted in EcoRI-SalI digested pSVSport-rNIS, and then in KpnI-NotI digested pShuttle.

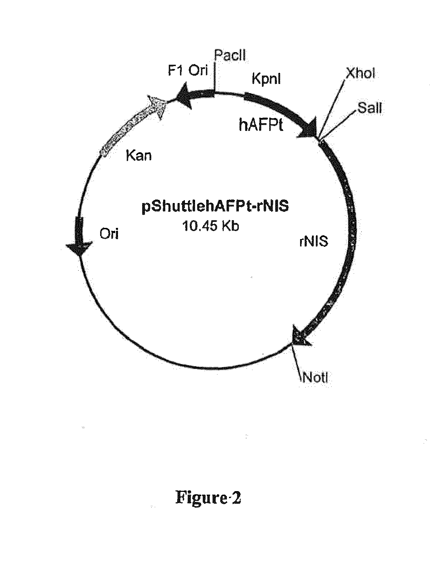

To generate pShuttle hAFPt-rNIS (Figure 2), the KpnI-XhoI 0.8 Kb fragment with a human alpha-fetoprotein (hAFP) enhancer elements was released from pGL3-hAFPt, and inserted in KpnI-SalI digested pShuttle-rNIS.

### Example 3 : Construction of the recombinant viral vectors.

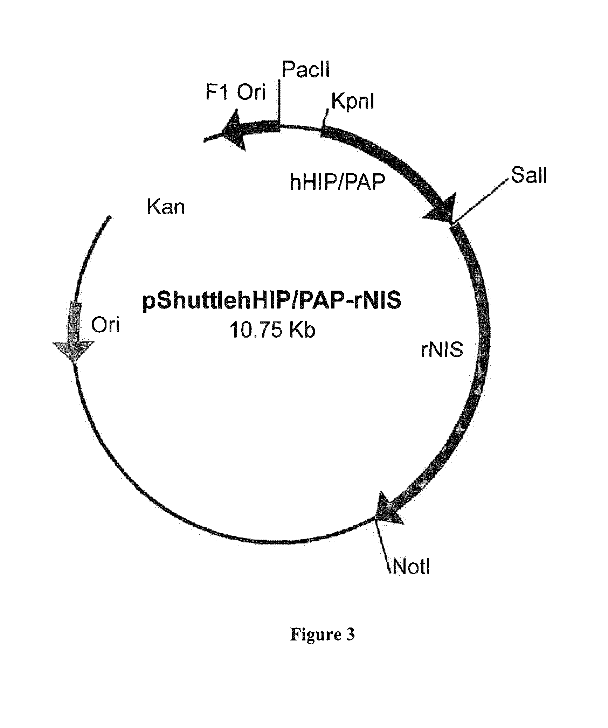

The pShuttle hHIP-(Figure 3), rHIP- rAFPwt- (Figure 1), and hAFPt-rNIS (Figure 2) were totally sequenced.

The generation of recombinant adenoviral plasmids by homologous recombination in E. Coli using the pAdEasy system as well as the production of adenoviruses were performed by the Vector Development Lab of Nantes following the method explained by He et al. (P.N.A.S, 1998; 95. 2509-14).

All recombinant adenoviruses were isolated from a single plaque, expanded in 293 cells, and purified by double caesium chloride centrifugation. The purified virus was stored in aliquots at -80°C. The virus titre was determined by plaque assay.

### Example 4 : In vitro and in vivo anti-cancer activity of the nucleic acids according to the invention.

### A. Material and Methods

### A.1. Luciferase assays.

HepG2 cells were seeded at 5x10⁶ cells in 10 cm Petri dishes. The next day, these cells were co-transfected with 10 µg of each reporter plasmid and 1 µg of pCH110 vector encoding the β-galactosidase gene using the calcium phosphate DNA coprecipitation method.

The β-galactosidase cotransfection was used as an internal control for normalizing transfection efficiency. Control plasmids were included, namely pCMV-Luciferase and pGL3-GFP 12h after transfection, IL-6 (100 units/mL) and dexamethasone (100 nM), alone or in combination, were added to the culture medium. 48 h after transfection, the cells were rinsed twice with phosphate-buffered saline (PBS) and scrapped on ice.

Cell extracts were prepared for the luciferase activity assay in isotonic buffer (20 mM Tris-HCl, pH 7.5, 137 mM NaCl, 1% Nonidet P-40) containing protease inhibitors. 50 µL of the supernatant were incubated with luciferine, and luciferase activity was measured using a luminometer. Promoter activity was expressed as luciferase activity normalized by beta-galactosidase activity.

### A.2. Ce// lines.

The human hepatoma HepG2, the human colon adenocarcinoma HT29 and the mammary carcinoma T47D cells were cultured in a 50/50 mix of Dulbecco's modified Eagle Medium glutamax (DMEM)/F12K Ham's (Invitrogen Corporation, Carlsbad, CA) supplemented with 10% foetal calf serum (FCS).

The human mammary adenocarcinoma cells MCF7 and the rat hepatoma BCXL3 were maintained in DMEM supplemented with 10% FCS. The AML12 (alpha mouse liver 12) cell line was maintained in F12K Ham's medium supplemented with 5 µg/mL insulin, 5 µg/mL transferrin, 40 ng/mL dexamethasone and 10% FCS.

Human primary adult hepatocytes were derived from liver pieces obtained after partial hepatectomy from liver metastasis and isolated using a collagenase H (Roche Molecular Biochemicals) perfusion (500 g/mL, 2.4 mg/mL CaCl₂ in HEPES buffer, pH 7.4) preceded by extensive washing of the liver tissue with HEPES/ ethylenediaminetetraacetic acid (EDTA) buffer pH 7.4 using a catheter inserted into the vessels on the cut surface of the resected fragment. The flow rate of the perfusion ranged from 15 to 18 mL/min. Cells were washed twice using soft centrifugation (100g for 2 min) and hepatocytes were separated from non parenchymatous cells using Percoll fractionation (30% isotonic Percoll solution). Viable cells were determined by Trypan blue exclusion and grown in complete William's medium (1 mg/mL bovine serum albumin, 25 nmol/L dexamethasone and 5 µg/mL bovine insulin) supplemented with 10% FCS.

### A.3. In vitro iodine uptake and efflux experiments.

The day before infection, 3x10⁵ cells were seeded into 24-well dishes. Cells were incubated with liver tumour specific NIS recombinant viral vectors or control vectors (i.e. empty vector Ad-DL324 and AdCMV-NIS) at different multiplicity of infection (10, 50, 100 and 200 infectious particles per cell) for 24 h. For HIP-NIS adenoviruses-infected cells, forty-eight hours after infection, IL-6 (100 units/mL) and dexamethasone (100 nM), alone or in combination, were added to the culture medium. Iodine uptake and efflux studies were then performed as previously described (Faivre *et al.,* 2004).

Briefly, cells were washed twice in B-Hank's Balanced Salt Solution (B-HBSS, Life Technologies, Inc), and then incubated for 60 min at 37°C in radioactive B-HBSS containing 1µCi (37 kBq) of ¹²³I and 0.5 µM of Nal (Fluka, Sigma-Aldrich) per well. At determined times, the cells were washed once with cold B-HBSS and then incubated for 20 min in 1 mL of cold absolute ethanol.

In iodine efflux studies, cells were incubated in the same radioactive medium as in uptake experiments for 60 min. The radioactive medium was then removed. The cells were washed once with B-HBSS, and 0.5 ml iodide-free B-HBSS was added. At the indicated times, the B-HBSS was removed, and the cells were incubated in cold absolute ethanol. The intra-cellular radioactivity was quantified using a well gamma-counter. The number of cells was determined as the average cell content of three wells. For NIS inhibition studies, NaCl0₄ was added at a concentration of 30 µM.

### A.4. Animals.

Male Wistar rats (Charles River, France) were treated in accordance with European Union regulations on animal care. Hepatocellular carcinoma was induced in 6 week-old male Wistar rats weighing 150-180 g by the daily intake of diethylnitrosamine (Sigma-Aldrich, St-Louis, MO) in their drinking water (100 mg/L) for 8 weeks. The rats had received thyroxin (T4) supplementation (50 µg/L) in their drinking water to reduce unwanted thyroid iodine uptake.

### A.5. In vivo Gene Transfer.

Rats were anaesthetized with 1 mL/kg of a 70:30 mix ketamine/xylazine (Imalgene 1000, Merial, Rompun, Bayer AG), and then laparotomized. The efficacy of gene transfer was evaluated for three routes of administration: intraportal, selective injection through the hepatic artery and direct injection into the tumour. Intraportal or Intra Hepatic Artery adenovirus vector expressing NIS was administered to different batches of rats (healthy, and DEN-treated rats bearing tumour nodules with a diameter smaller than 10 mm). The rNIS vector was directly injected into 5 to 10 mm diameter tumour nodules in DEN-treated rats using a 30-gauge needle. Different amounts of virus were evaluated ranging from 5x10⁹ to 10¹¹ infectious particles injected per rat.

### A.6. In vivo iodine uptake experiments

### A.6.1. Planar scintigraphic imaging.

Na ¹²³I (Schering AG, Berlin, Germany) was injected intraperitoneally (6 to 12 MBq per rat). From two to four rats were imaged simultaneously using a high-resolution parallel collimator of either low- or high-energy for ¹²³I or ¹³¹I imaging, respectively. Scintigraphic 256x256 images were obtained using a DSX gamma camera (GE). An aliquot of the tracer administered was also imaged as an internal standard. The same procedure was applied serially for kinetic studies. We obtained higher-resolution images by using a 4 mm hole pinhole collimator and increasing acquisition times and numerization. After this image acquisition, each rat was injected intraperitoneally with 50 mg of sodium perchlorate, a potent inhibitor of active iodide transport, and images were acquired at 1 min intervals for 60 min.

### A.6.2. Single-photon emission computed tomography and CT (SPECT/CT) imaging.

[^{99m}Tc] pertechnetate was injected intravenously (37 MBq per rat). Fifteen minutes after injection, the rats were placed vertically with liver positioned in front of the acquisition window. The SPECT acquisition was performed using a 4 mm-thick x 120 mm-diameter CsI(Na) continuous crystal and a position-sensitive photomultiplier, leading to an intrinsic spatial resolution of 2 mm and a field of view of 10 cm. The CT system comprises a scintillating sheet coupled with a lens to a cooled CCD, leading to a 250 µm spatial resolution. SPECT/CT (Biospace Mesures, Paris) performs real time imaging: simultaneous SPECT and CT data acquisition enables tomographic reconstruction of the SPECT image, the CT image and registration of both images during acquisition. SPECT and CT images were acquired during 40 min. Reconstructed images were analyzed using the Amira 3.1.1 analysis tool (Mercury Computer Systems, Inc).

### A.6.3. Image quantification.

We used the region of interest (ROI) method with appropriate background correction to calculate specific activities. Absolute effective 120 min-uptakes in a given organ were computed as the background corrected counts within this organ divided by the counting efficiency of the collimated system and the administered activity at time 0. Physical decays are of 13.2 h for ¹²³I and 8.05 d for ¹³¹I, respectively. Biological uptakes were computed as effective uptakes divided by the isotopic physical decay corresponding to the time intervals between intraperitoneal injection and imaging. Normalized corrected count rates and the activity of a specific ROI were measured in the images from 120 min onwards and organ half-lives were calculated using a mono-exponential fit.

### A.6.4. Western blot analysis.

Cellular protein extracts were prepared the day after viral infection. Cells were scrapped and homogenized in a lysis buffer containing 50 mmol/L Tris pH 8.0, 150 mmol/L NaCl, 1% Nonidet P-40, 100 µg/mL phenylmethylsulfonylfluoride, 1 µg/mL aprotinin, and 1 µg/mL leupeptin.

The lysates were then centrifuged at 12,000 g for 10 min at 4°C and solubilized in 2X Laemmli buffer followed by heating 10 minutes at 96°C. Protein concentration was determined by the Bradford method. Proteins were separated using a SDS-9% polyacrylamide gel electrophoresis and transferred to nitrocellulose. Western blot analysis was performed by incubating the filter with an anti-rNIS peptide polyclonal antibody pAb-1 0 (1:2000) in Tris-buffered saline diluted in 0.1% Tween-20 containing 5% nonfat dry milk for 1 hour at room temperature, followed by incubation with horseradish peroxidase-conjugated goat anti-rabbit IgG (1:1000,) for 1 h at room temperature.

Detection was performed using the enhanced chemiluminescence Western blot detection system (Super Signal, Pierce). For deglycosylation assays, proteins were denatured in the denaturing buffer (0.5% SDS and 1% ß-mercaptoethanol) for 30 min at 37 C. Denatured proteins were incubated at 37°C overnight with peptidyl:N-glycosidase F (PNGase F; New England Biolabs, Inc., Beverly, MA) at the concentration of 500U per µg of protein.

### A.6.4. Histology, immunofluorescence, micro-autoradiography.

Formalin-fixed tissue sections were embedded in paraffin, stained with H&E and examined under a light microscope. For immunofluorescence, cryosections (4-µm) were fixed in 4% paraformaldehyde for 15 min and incubated for 1 hour with the primary polyclonal antibody to NIS diluted at 1:200. Fluorescence was examined by UV-fluorescence microscopy. ¹³¹I labeled liver sections were analyzed with a Micro-Imager (24x32 mm² field of view and 25µM resolution, Biospace Mesures).

### A.6.5. In vitro uptakes.

Organ samples were weighed, radioactivity was measured in a well gamma-counter and the results expressed as a percentage of the injected dose per gram of tissue (%ID/g). Auto-absorption can be discounted for the volumes and gamma emitters employed.

### A.7. Statistics.

Statistical comparisons were performed using variance analysis and a PLSD Fischer test at risk 5% (StatView 5.0 software SAS system).

### B. Results

The aim of the experimental procedures performed in this example was to make the destruction of hepatic tumour by internal radiotherapy possible at relatively low global irradiation dose in order to avoid side effects such as myelo-ablation. The purpose was thus to define recombinant vectors, either viral or non viral, capable of inducing, after transfer, an accumulation of a radioisotope specifically in liver tumour cells through the expression of the Sodium Iodide Symporter (NIS) in the tumour cells.

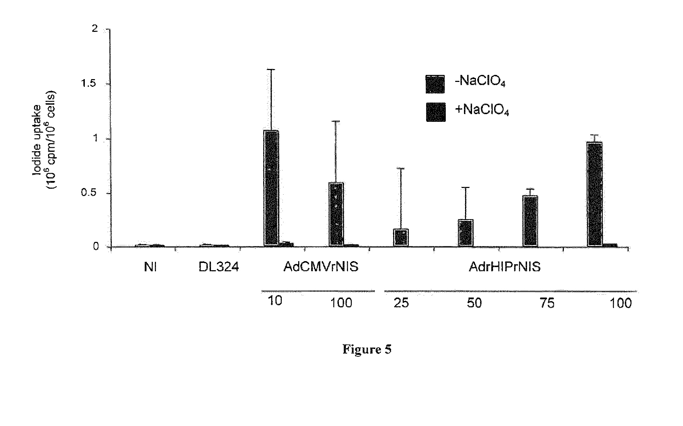

As shown in Figure 5, the rat HIP/PAP regulatory sequence successfully induces expression of NIS in human tumour hepatocytes.

Further, anti-NIS immunofluorescence assays using HepG2 cells transfected with the adenoviral vector AdrHIPrNIS showed that NIS was expressed at the plasma membrane level of transfected cells.

As shown in Figure 6, the human HIP/PAP regulatory sequence also successfully induces expression of NIS in human tumour hepatocytes.

Further, as shown in Figure 7, the rat HIP/PAP regulatory sequence induces a strong *in vivo* expression of NIS, specifically in liver tumour nodules.

Still further, as shown in Figure 8, both the human and rat AFP-derived regulatory sequences induce expression of NIS in human tumour hepatocytes.

Additionally, when transfecting human cells with a viral vector comprising a GFP DNA sequence operably linked to a human AFP-derived regulatory sequence, it was shown that GFP was exclusively and strongly expressed in human hepatic cancer cells.

Further, anti-NIS immunofluorescence assays using HepG2 cells transfected with the adenoviral vector AdAFPrNIS showed that NIS was expressed at the plasma membrane of transfected cells.

The successful targeting of NIS expression will allow one to irradiate strongly the sole tumours while minimising the whole body irradiation dose.

The major obstacle that had to be surmounted was the fact that the available tumour specific promoters that regulate the expression of the potential therapeutic genes are in small number and have weak transcriptional activities. Therefore, the NIS protein expression modulated by these former promoters was not sufficient for the uptake of cytolytic concentrations of radioisotopes.

According to the invention, this problem has been solved by selecting truncated regulatory sequences of the liver tumour specific genes AFP and HIP/PAPI for the construction of NIS recombinant vectors.

According to the experimental results of this example, it has been shown *in vivo* that these vectors exhibit a sufficiently high transcriptional activity for NIS in liver tumours of HCC-bearing animal models, as demonstrated by the clear inhibition of tumour growth after 131I therapy.

Thus, it has been herein experimentally performed the following:
- construction of truncated regulatory sequences of alphafetoprotein (AFP), and of the Hepatocarcinoma Intestine Pancreas/Pancreatitis Associated Protein I (HIP/PAP I) genes fused to a reporter gene, and selected among these constructs the ones that exhibited the highest transcriptional activities;
- construction and testing by sequencing, rat NIS recombinant adeno- and lentiviruses harboring the selected regulatory sequences AFP and HIP/PAPI;
- demonstration *in vitro* that, after infection by AFP-NIS and HIP/PAPI-NIS, the NIS protein expression is strong and correctly targeted to the plasma membrane of transformed hepatic cells, whereas it is not expressed in primary normal hepatocytes;
- demonstration *in vitro* that the uptake levels of iodine in cells infected by AFP-NIS and HIP/PAPI-NIS are strong (comparable to those induced by CMV-NIS);
- demonstration *in vivo,* by serial radioisotope imaging in HCC-bearing animals, that radioiodide is strongly taken-up by hepatic tumour nodules, and not by the surrounding non-tumour tissues, which indicates a strong functional NIS expression in the sole tumor hepatocytes thanks to the regulatory sequences AFP and HIP/PAPI;
- demonstration *in vivo* of a significant tumour regression in HCC-bearing animals in response to a ¹³¹I radiotherapy following AFP-NIS or HIP/PAPI-NIS transduction.

According to the experimental results which have been herein obtained, it was shown that local conditions are very different in the organs from what they are in grafted tumours, in particular because of the dense vascularisation, the fibro-inflammatory peri-tumour reaction, and the underlying diseases (cirrhosis and chronic hepatitis) existing in liver cancers. Therefore it would be irrelevant to extrapolate to patients the dramatic efflux of iodide noted in grafted tumours. The inventors have shown that the long-lasting retention of radioiodide in liver tumours, which is a necessary condition for the efficiency of radiotherapy, is the result of a dynamical process of uptake, efflux and re-uptake, the latter being favoured by a high concentration of the NIS protein at the plasma membrane of the tumour hepatocytes, induced by the AFP-NIS or HIP/PAP-NIS vectors, and by the high hepatic blood flow, which allows the rapid recycling of plasmatic radioiodine to the liver.

**Table of the sequences**

| **SEQ ID N°** | **Type** | **Description** |
|---|---|---|
| 1 | nucleic acid | fragment derived from human AFP promoter |
| 2 | nucleic acid | fragment derived from human AFP promoter |
| 3 | nucleic acid | fragment derived from human AFP promoter |
| 4 | nucleic acid | human HIP/PAPI promoter |
| 5 | nucleic acid | rat PAPI promoter |
| 6 | nucleic acid | fragment derived from rat AFP promoter] |
| 7 | nucleic acid | fragment derived from rat AFP promoter |
| 8 | nucleic acid | fragment derived from rat AFP promoter |
| 9 | nucleic acid | human AFP-derived regulatory sequence |
| 10 | nucleic acid | human AFP-derived regulatory sequence |
| 11 | nucleic acid | human HIP/PAPI regulatory sequence |
| 12 | nucleic acid | human HIP/PAPI regulatory sequence |
| 13 | nucleic acid | human HIP/PAPI regulatory sequence |
| 14 | nucleic acid | human HIP/PAPI regulatory sequence |
| 15 | nucleic acid | rat AFP-derived regulatory sequence |
| 16 | nucleic acid | rat AFP-derived regulatory sequence |
| 17-20 | nucleic acid | primers |

## Claims

1. A nucleic acid for expressing a polynucleotide of interest specifically in mammalian cancer cells, the said nucleic acid comprising:
(a) a regulatory polynucleotide that drives the expression of the said polynucleotide of interest in mammalian cancer cells, said regulatory polynucleotide comprising, from 5'-end to 3'-end :
- a first nucleic acid having at least 90% nucleotide identity with a nucleic acid selected from the group consisting of SEQ ID N°1 and SEQ ID N°2; and
- a second nucleic acid having at least 90% nucleotide identity with the nucleic acid of SEQ ID N°3;
- the said first and second nucleic acids being linked directly to each other, or
- the said first and second nucleic acids being linked indirectly to each other through a spacer nucleic acid having from 1 to 3000 nucleotides in length, and
(b) the polynucleotide of interest, said polynucleotide of interest inducing anti-cancer activity when expressed under the control of the regulatory polynucleotide.

2. The nucleic acid according to claim 1, wherein the said regulatory polynucleotide comprises, from 5'-end to 3'-end:
- a first nucleic acid having at least 90% nucleotide identity with a nucleic acid selected from the group consisting of SEQ ID N°1 and SEQ ID N°2;
- a spacer nucleic acid having a nucleotide length ranging from 1 to 3000 nucleotides, and
- a second nucleic acid having at least 90% nucleotide identity with the nucleic acid of SEQ ID N°3.

3. The nucleic acid according to anyone of claim 1 or 2, wherein the said regulatory polynucleotide further comprises one or both of the following additional nucleic acids:
- a 5'-additional nucleic acid that is linked to the 5'-end of the said first nucleic acid,
- a 3'-additional nucleic acid that is linked to the 3'-end of the said second nucleic acid,
said additional nucleic acids having a nucleotide length ranging from 1 to 1500 nucleotides.

4. The nucleic acid according to claim 1 , wherein the said regulatory polynucleotide has at least 90% nucleotide identity with a nucleic acid selected from the group consisting of SEQ ID N°9 and SEQ ID N° 10.

5. The nucleic acid according to any one of claims 1 to 4, wherein said polynucleotide of interest encodes a protein.

6. The nucleic acid according to any one of claims 1 to 5, wherein said polynucleotide of interest encodes the sodium iodide symporter (NIS) protein.

7. The nucleic acid according to any one of claims 1 to 6, which is inserted in an expression vector.

8. A recombinant vector comprising inserted therein the nucleic acid according to any one of claims 1 to 7.

9. The recombinant vector according to claim 8, which is replicable in bacterial cells.

10. The recombinant vector according to claim 8, which is replicable in mammalian cells.

11. The recombinant vector according to claim 8, which consists of a recombinant viral vector.

12. The recombinant vector according to claim 8, wherein said vector is selected from the group consisting of recombinant adenovirus and recombinant lentivirus.

13. A recombinant host cell, which has been transfected with the recombinant vector according to any one of claims 8 to 12.

14. The recombinant host cell according to claim 13, wherein said recombinant cell is selected from the group consisting of bacterial cell and mammalian cell.

15. A pharmaceutical composition comprising:
(i) an active ingredient selected from the group consisting of :
- the nucleic acid according to any one of claims 1 to 7; and
- the recombinant vector according to any one of claims 8 to 12; and
(ii) one or more pharmaceutically compatible excipients.


SEQUENCE LISTING
<110> INSERM (Institut National de la Santé et de la Recherche Médicale)
<120> Nucleic acids for expressing a polynucleotide of interest in ammamlian
cancer cells
<130> T892EP - INSERM
<160> 20
<170> PatentIn version 3.1
<210> 1
<211> 533
<212> DNA
<213> Homo sapiens
<400> 1
aaaatctaat tacttttagc ccagtgctca tcccacctat ggggagatga gagtgaaaag 60
ggagcctgat taataattac actaagtcaa taggcataga gccaggactg tttgggtaaa 120
ctggtcactt tatcttaaac taaatatatc caaaactgaa catgtactta gttactaagt 180
ctttgacttt atctcattca taccactcag ctttatccag gccacttatt tgacagtatt 240
attgcgaaaa cttcctaact ggtctcctta tcatagtctt atcccctttt gaaacaaaag 300
agacagtttc aaaatacaaa tatgattttt attagctccc ttttgttgtc tataatagtc 360
ccagaaggag ttataaactc catttaaaaa gtctttgaga tgtggccctt gccaactttg 420
ccaggaattc ccaatatcta gtattttcta ctattaaact ttgtgcctct tcaaaactgc 480
attttctctc attccctaag tgtgcattgt tttcccttac cggttggttt ttc 533
<210> 2
<211> 807
<212> DNA
<213> Homo sapiens
<400> 2
actgtcaaat aagtggcctg gataaagctg agtggtatga atgagataaa gtcaaagact 60
tagtaactaa gtacatgttc agttttggat atatttagtt taagataaag tgaccagttt 120
acccaaacag tcctggctct atgcctattg acttagtgta attattaatc aggctccctt 180
ttcactctca tctccccata ggtgggatga gcactgggct aaaagtaatt agattttaag 240
gaacaagagg accctatgtg ctacaggaca cattttagga aacattccag gcaaaactcc 300
ccggtttgta tgttgaaacg tctttaagcc ctccaagtgg agacgttgat gtgcatttga 360
atactacttt ttatcttctg gaacaagatt gaggctagag atagatattt gggaatcata 420
agcttgcaga cagttattgt agccatgtga gtggttgaga aaaatagata gcagtgaaaa 480
gagaaaccat gaagaacagc aaattttcca agacaagcaa taaagaggag ctttgaacca 540
gagtttgtat actaacagca aacaatcctc aatctatatg tgttattgct caaagtttag 600
tcctaagtac agagttcaca gcaatgcttt tgcattgatg taattctgat tccgtttact 660
ttttcataag tctgtgaagc cactcagaag caacatggaa cagtaaactg aactttggta 720
ttagaaagat tcaaaactaa tttaaatcta attgtcttat ggtcaattat tagctgtatg 780
acaggcgaat tctagcccct agatttt 807
<210> 3
<211> 213
<212> DNA
<213> Homo sapiens
<400> 3
ctgccccaaa gagctctgtg tccttgaaca taaaatacaa ataaccgcta tgctgttaat 60
tattggcaaa tgtcccattt tcaacctaag gaaataccat aaagtaacag atataccaac 120
aaaaggttac tagttaacag gcattgcctg aaaagagtat aaaagaattt cagcatgatt 180
ttccatattg tgcttccacc actgccaata aca 213
<210> 4
<211> 2426
<212> DNA
<213> Homo sapiens
<400> 4
ctgtgtcatc cctaagaagt agatttttat ataactcaaa accagctcaa gaaatataat 60
atttaaacaa acttctcagt gcttggttaa taagcattgt tatgaagaaa caatgaaaaa 120
taaaacaaaa acaactcaac tttagtgagt ttagtgagtg tcattgcaaa gaaaatttca 180
cattgcactt agggatttta taaccttaag gcttattaca aaggtcacac tatttcaatt 240
aagaacaaaa gtgagtttta ataataaact gtcagcattt caaatcttct ctcctaggta 300
tcttgaaata tacaacatat tgttgttaac tatagtcacc ctatgtgcta tcaaaagcta 360
gaacatattc ctcctatcta gctgtatgtt tgtacgatga actaaactct tttcatctcc 420
cctcacccgt ctcagcctct ggtaactatt ctaccctctg gttaccttct accccgtacc 480
tccatgtgtt tacatattgt tgtataaatt ttgtggaaaa aatccttttt taacaaaata 540
atctgctggc tatatttctt ctcatattat ttgaggaatt gcattgaatt ttgggtactg 600
aattttgagg acgtgaatta aggatgagct acatctcatg acctcgtcac agacccctac 660

tataaacatc tgcatacaag ttggcatatt acaacaatag catgagagtc agggtctgta 720
gaaattccca gcctaacttt ttcctggact ggccaaactg aacctgaacc agtgtatggg 780
caatgtagta tgcttgtcag aggcaccaaa ctgctaactc aaaaataaac atgactaaaa 840
gaacttctcc cagtataccc atatgaagct gaccatttgc ccagtaagaa tgcaatgaaa 900
agggctttta atgtacagcc aaatggcagt ccagcaagga agctcatgag caacctacaa 960
gtagtgagca ttttgatgta atagagactt acatgcaatt cagaattatg tttacaacat 1020
cacaaaacaa aaatttagag ataggctctc actctgttgc ctaagctgga gtgcagtggg 1080
atgatcataa cttactgcag ccttgaactc ctgggttcaa ctgaaggtcc tcctacctca 1140
gcctgctgag tagctaggac cacaagcaca caccaccgca actggcttaa attaaaatat 1200
aaattgtaga gatagggtct taatgtgttg cccaggctgc tcttgaactc cttgcttcag 1260
gtgatcctcc cacctcagcc tctcaaagtg ctgggattat agacctgagc cacagcacct 1320
ggccaactga cctatgattt tacacaatgg ctgctcttcc cttctttaac tattattcat 1380
tcttctttga tcctcattat ttgactgtag tccttcttat gtcttgtttt ccttcattac 1440
ctcttattct atcacattgc cattgtcatt ctccactggg gaagctcttt cttgctgaag 1500
actggaaaga caagtccatt cacctgattt tctgtaagat tgtggctcat gtattgactt 1560
gtcagacaat tctgaagttt catcaaaatt agctatcatg cttgcataat ggccctgaac 1620
cctcactcct actcttagct tcagtaccat ctatgtcctc aactgtccat gatacttata 1680
attcccgtaa atcttcactt aacacctaac atttatttaa tcttactagg caaggtaata 1740
agaaatacat aggtttgcct ccagaagtgg gttcttaaga aacccaccag aggaactcct 1800
ctttcagatg tccacattag aagatttcat atcacatttg gtgccacagg cctttgacaa 1860
ggaggatgca gaggaaaaag caaacttcac ctcttcctag ggaaagtgtt ggcctgccaa 1920
caggaaagag gcaacatctg ggaaaatccc cagtctttgc caggaagagt ccatgccaac 1980
cccaccccat gacccctgtc ctgcctactc attgtcactc ttcactccaa tgtccctccc 2040
ccagatcctc ttataaaatc ccactctttc ctgaccagac aaaccatacc atatcccacc 2100
agagaggtaa gtgggagctg agagaagatg agacccaggg aggagctact gcacatgaca 2160
caggagaata catgggaggg tcccttcctc agggagcaca ggaactctga gactcagcaa 2220
gggtgtcctg ggagggctcg gggatgggag agtacacaga ttcacaactc attcagaact 2280
gtagaagatg atggatgtga ccaagatcac tttagtccta ggggactaga gaaggaaaat 2340
gacatgaggc agtggggtat ctgtgtgttc tcccactgac cacgctttct ttagtgactc 2400
ctgattgcct cctcaagtcg cagaca 2426
<210> 5
<211> 1253
<212> DNA
<213> Rattus sp.
<400> 5
ctgcagattt tccagttagt cataaagact atcaaacaaa tgtacacaag atttgatatt 60
tagttcagtt aactaatttt ctgctaacaa agtcaaatgt atttcaaagc catgatgtct 120
tcttgcgcat gtgctaatgt ggaagcatcc caaggttttg cccacagcag ggtttctgtc 180
ctcagacttc tctctgatct tgctttgaga taggctgcta ataagatacc cagattggcc 240
ttgaacttat tcttccacat agacaggtcg tgcctacatt gcaacccttc tacttcagca 300
tcctgagtag tttggattgc aaatctgtgc tccagaacca gacaatggct atttgctatt 360
tataagtata caagaagaac acaggctggt gacctggaaa gctatggaac cttttttggt 420
accatgagta caaatgtcac agtgtgaaaa cagaaaagga acactcacta tgaaatgaag 480
ggtagacata cacagttatt gtaaaagggt ccagaagatt actggtccta tgagatctca 540
gtctgtacat gatatccaag gcagaaaatt accaagctgg acagaggaca atgaatgaga 600
cttattctta acctcagaga aatgtatgta aggtaagttg gttgatatga tttttcttaa 660
ggtttgtgca agaatgttta cttaataaaa gctaacttta caaaatgata tgaagcttta 720
tcaacatatc taaaatcatc atttctttct ttagtatgaa tatataactt tgaatcattt 780
aaattacacc tacaattttt aatccactca attcttacta atttttagac ttccagatga 840
atttcaagca atctgtataa aagtgagact ttttaaattg attctaaatg cactcagatt 900
tatttttctt aacttatcag gtaataaaac tttaaaacta tgtcacagaa aaatgtgttt 960
cccaatgcca ccattttttc cttgtgtttc ccagaacaga gtatctggaa aagggtgtgg 1020
agggttcaaa cttcactcac acctgaggta aagcagggct gctaacagga aagaggtaat 1080
atttatgaca attatcagtt gttaccagaa aagtcacctg ctctttcttg gaaactcttt 1140
gagccaatgc caagtgcttc ccatcatcct aggctgcata cgcattccag ttatttacta 1200
tgatgcccct ccctcatgtc ccgttataaa aacacagcca tctctgtcct cac 1253
<210> 6
<211> 746
<212> DNA
<213> Rattus sp.
<400> 6
actagtttat ctaaccctcg tttattaaaa aggatattaa ttttcgtaac tataattttt 60
atatgttggg agtaaaacca ttttgagtgt tttgtccaat gtcacctgac cgacagtttg 120
aatagtcggg ggtagagcct ttcgtatact aaagtccagt ttgtttaacc atattgcttc 180
agtggggttt catgggctca ggaagtaacg aatgaaccag acatagagct atgaaaggta 240

tgtggtgcga gctcagccct tgcgacaaag ctttgagcaa cagcccgcgt gggcttaggg 300
ttgtttgcag ttggtgttag agacctcaca caaagtcatg tggcagataa cccggaggca 360
aaattcaaac ccagtcgcca tatgctcatg tttaacggtg accctgtgca cctttctgat 420
cacatgcttt ggaattgcaa agatctcccc acaaggcaga gtgcagagag aattaaggat 480
gacataaccc tgtgggctgg gctgatctgg gctgctcctc ttggcttagg tgtagaagca 540
tagcagtgaa ttggtgactg atataacgtg tatttattat ctatagtttt gtgtgtgtgt 600
gtgtgtgtgt gtgtgtgtgt gtgtgtgtgt gtgtgtatga tcatatttac acatgattca 660
tctagccttt atgaaaggat gatgaaacca gacatttagc cttgcggtta catgcatact 720
agcaagaaac tcgatatagg atcttt 746
<210> 7
<211> 746
<212> DNA
<213> Rattus sp.
<400> 7
tgatcaaata gattgggagc aaataatttt tcctataatt aaaagcattg atattaaaaa 60
tatacaaccc tcattttggt aaaactcaca aaacaggtta cagtggactg gctgtcaaac 120
ttatcagccc ccatctcgga aagcatatga tttcaggtca aacaaattgg tataacgaag 180
tcaccccaaa gtacccgagt ccttcattgc ttacttggtc tgtatctcga tactttccat 240
acaccacgct cgagtcggga acgctgtttc gaaactcgtt gtcgggcgca cccgaatccc 300
aacaaacgtc aaccacaatc tctggagtgt gtttcagtac accgtctatt gggcctccgt 360
tttaagtttg ggtcagcggt atacgagtac aaattgccac tgggacacgt ggaaagacta 420
gtgtacgaaa ccttaacgtt tctagagggg tgttccgtct cacgtctctc ttaattccta 480
ctgtattggg acacccgacc cgactagacc cgacgaggag aaccgaatcc acatcttcgt 540
atcgtcactt aaccactgac tatattgcac ataaataata gatatcaaaa cacacacaca 600
cacacacaca cacacacaca cacacacaca cacacatact agtataaatg tgtactaagt 660
agatcggaaa tactttccta ctactttggt ctgtaaatcg gaacgccaat gtacgtatga 720
tcgttctttg agctatatcc tagaaa 746
<210> 8
<211> 205
<212> DNA
<213> Rattus sp.
<400> 8
tctgaagtgg tctttgtcct tgaacataag atagaagtga cccctgtgct gttaattatt 60
ggcaaattgc ctaacttcaa cataaggaaa aaaagtaaca tgtttgccca ctgaaggtta 120
ctagttaaca gacatcactt aaaaaggata taaaagaact tcagcgctac tgctcacagt 180
atccggcttc taccactgtc tggga 205
<210> 9
<211> 1020
<212> DNA
<213> Homo sapiens
<400> 9
actgtcaaat aagtggcctg gataaagctg agtggtatga atgagataaa gtcaaagact 60
tagtaactaa gtacatgttc agttttggat atatttagtt taagataaag tgaccagttt 120
acccaaacag tcctggctct atgcctattg acttagtgta attattaatc aggctccctt 180
ttcactctca tctccccata ggtgggatga gcactgggct aaaagtaatt agattttaag 240
gaacaagagg accctatgtg ctacaggaca cattttagga aacattccag gcaaaactcc 300
ccggtttgta tgttgaaacg tctttaagcc ctccaagtgg agacgttgat gtgcatttga 360
atactacttt ttatcttctg gaacaagatt gaggctagag atagatattt gggaatcata 420
agcttgcaga cagttattgt agccatgtga gtggttgaga aaaatagata gcagtgaaaa 480
gagaaaccat gaagaacagc aaattttcca agacaagcaa taaagaggag ctttgaacca 540
gagtttgtat actaacagca aacaatcctc aatctatatg tgttattgct caaagtttag 600
tcctaagtac agagttcaca gcaatgcttt tgcattgatg taattctgat tccgtttact 660
ttttcataag tctgtgaagc cactcagaag caacatggaa cagtaaactg aactttggta 720
ttagaaagat tcaaaactaa tttaaatcta attgtcttat ggtcaattat tagctgtatg 780
acaggcgaat tctagcccct agattttctg ccccaaagag ctctgtgtcc ttgaacataa 840
aatacaaata accgctatgc tgttaattat tggcaaatgt cccattttca acctaaggaa 900
ataccataaa gtaacagata taccaacaaa aggttactag ttaacaggca ttgcctgaaa 960
agagtataaa agaatttcag catgattttc catattgtgc ttccaccact gccaataaca 1020
<210> 10
<211> 4185
<212> DNA
<213> Homo sapiens
<400> 10
ggatagatca ttttcatgat atatgagaaa aattaaaaat cagattgaat tatttgcctg 60
tcatacagct aataattgac cataagacaa ttagatttaa attagttttg aatctttcta 120

ataccaaagt tcagtttact gttccatgtt gcttctgagt ggcttcacag acttatgaaa 180
aagtaaacgg aatcagaatt acatcaatgc aaaagcattg ctgtgaactc tgtacttagg 240
actaaacttt gagcaataac acatatagat tgaggattgt ttgctgttag tatacaaact 300
ctggttcaaa gctcctcttt attgcttgtc ttggaaaatt tgctgttctt catggtttct 360
cttttcactg ctatctattt ttctcaacca ctcacatggc tacaataact gtctgcaagc 420
ttatgattcc caaatatcta tctctagcct caatcttgtt ccagaagata aaaagtagta 480
ttcaaatgca catcaacgtc tccacttgga gggcttaaag acgtttcaac atacaaaccg 540
gggagttttg cctggaatgt ttcctaaaat gtgtcctgta gcacataggg tcctcttgtt 600
ccttaaaatc taattacttt tagcccagtg ctcatcccac ctatggggag atgagagtga 660
aaagggagcc tgattaataa ttacactaag tcaataggca tagagccagg actgtttggg 720
taaactggtc actttatctt aaactaaata tatccaaaac tgaacatgta cttagttact 780
aagtctttga ctttatctca ttcataccac tcagctttat ccaggccact tatttgacag 840
tattattgcg aaaacttcct aactggtctc cttatcatag tcttatcccc ttttgaaaca 900
aaagagacag tttcaaaata caaatatgat ttttattagc tcccttttgt tgtctataat 960
agtcccagaa ggagttataa actccattta aaaagtcttt gagatgtggc ccttgccaac 1020
tttgccagga attcccaata tctagtattt tctactatta aactttgtgc ctcttcaaaa 1080
ctgcattttc tctcattccc taagtgtgca ttgttttccc ttaccggttg gtttttccac 1140
caccttttac attttcctgg aacactatac cctccctctt catttggccc acctctaatt 1200
ttctttcaga tctccatgaa gatgttactt cctccaggaa gccttatctg acccctccaa 1260
agatgtcatg agttcctctt ttcattctac taatcacagc atccatcaca ccatgttgtg 1320
attactgata ctattgtctg tttctctgat taggcagtaa gctcaacaag agctacatgg 1380
tgcctgtctc ttgttgctga ttattcccat ccaaaaacag tgcctggaat gcagacttaa 1440
cattttattg aatgaataaa taaaacccca tctatcgagt gctactttgt gcaagacccg 1500
gttctgaggc atttatattt attgatttat ttaattctca tttaaccatg aaggaggtac 1560
tatcactatc cttattttat agttgataaa gataaagccc agagaaatga attaactcac 1620
ccaaagtcat gtagctaagt gacagggcaa aaattcaaac cagttcccca actttacgtg 1680
attaatactg tgctatactg cctctctgat catatggcat ggaatgcaga catctgctcc 1740
gtaaggcaga atatggaagg agattggagg atgacacaaa accagcataa tatcagagga 1800
aaagtccaaa caggacctga actgatagaa aagttgttac tcctggtgta gtcgcatcga 1860
catcttgatg aactggtggc tgacacaaca tacattggct tgatgtgtac atattatttg 1920
tagttgtgtg tgtattttta tatatatatt tgtaatattg aaatagtcat aatttactaa 1980
aggcctacca tttgccaggc atttttacat ttgtcccctc taatcttttg atgagatgat 2040
cagattggat tacttggcct tgaagatgat atatctatat ctacatctat atctatatct 2100
atatctatat ctatatctat atatgtatat cagaaaagct gaaatatgtt ttgtaaagtt 2160
ataaagattt cagactttat agaatctggg atttgccaaa tgtaacccct ttctctacat 2220
taaacccatg ttggaacaaa tacatttatt attcattcat caaatgttgc tgagtcctgg 2280
ctatgaacca gacactgtga aagcctttgg gatattttgc ccatgcttgg gcaagcttat 2340
atagtttgct tcataaaact ctatttcagt tcttcataac taatacttca tgactattgc 2400
ttttcaggta ttccttcata acaaatactt tggctttcat atatttgagt aaagtccccc 2460
ttgaggaaga gtagaagaac tgcactttgt aaatactatc ctggaatcca aacggataga 2520
caaggatggt gctacctctt tctggagagt acgtgagcaa ggcctgtttt gttaacatgt 2580
tccttaggag acaaaactta ggagagacac gcatagcaga aaatggacaa aaactaacaa 2640
atgaatggga attgtacttg attagcattg aagaccttgt ttatactatg ataaatgttt 2700
gtatttgctg gaagtgctac tgacggtaaa ccctttttgt ttaaatgtgt gccctagtag 2760
cttgcagtat gatctatttt ttaagtactg tacttagctt atttaaaaat tttatgttta 2820
aaattgcata ctgctctttc attgaagaag ttttgagaga gagatagaat taaattcact 2880
tatcttacca tctagagaaa cccaatgtta aaactttgtt gtccattatt tctgtctttt 2940
attcaacatt ttttttagag ggtgggagga atacagagga ggtacaatga tacacaaatg 3000
agagcactct ccatgtattg ttttgtcctg tttttcagtt aacaatatat tatgagcata 3060
tttccatttc attaaatatt cttccacaaa gttattttga tggctgtata tcaccctact 3120
ttatgaatgt accatattaa tttatttcct ggtgtgggtt atttgatttt ataatcttac 3180
ctttagaata atgaaacacc tgtgaagctt tagaaaatac tggtgcctgg gtctcaactc 3240
cacagattct gatttaactg gtctgggtta cagactaggc attgggaatt caaaaagttc 3300
ccccagtgat tctaatgtgt agccaagatc gggaaccctt gtagacaggg atgataggag 3360
gtgagccact cttagcatcc atcatttagt attaacatca tcatcttgag ttgctaagtg 3420
aatgatgcac ctgacccact ttataaagac acatgtgcaa ataaaattat tataggactt 3480
ggtttattag ggcttgtgct ctaagttttc tatgttaagc catacatcgc atactaaata 3540
ctttaaaatg taccttattg acatacatat taagtgaaaa gtgtttctga gctaaacaat 3600
gacagcataa ttatcaagca atgataattt gaaatgaatt tattattctg caacttaggg 3660
acaagtcatc tctctgaatt ttttgtactt tgagagtatt tgttatattt gcaagatgaa 3720
gagtctgaat tggtcagaca atgtcttgtg tgcctggcat atgataggca tttaatagtt 3780
ttaaagaatt aatgtattta gatgaattgc ataccaaatc tgctgtcttt tctttatggc 3840
ttcattaact taatttgaga gaaattaatt attctgcaac ttagggacaa gtcatctctt 3900
tgaatattct gtagtttgag gagaatattt gttatatttg caaaataaaa taagtttgca 3960
agtttttttt ttctgcccca aagagctctg tgtccttgaa cataaaatac aaataaccgc 4020

tatgctgtta attattggca aatgtcccat tttcaaccta aggaaatacc ataaagtaac 4080
agatatacca acaaaaggtt actagttaac aggcattgcc tgaaaagagt ataaaagaat 4140
ttcagcatga ttttccatat tgtgcttcca ccactgccaa taaca 4185
<210> 11
<211> 233
<212> DNA
<213> Homo sapiens
<400> 11
gagcacagga actctgagac tcagcaaggg tgtcctggga gggctcgggg atgggagagt 60
acacagattc acaactcatt cagaactgta gaagatgatg gatgtgacca agatcacttt 120
agtcctaggg gactagagaa ggaaaatgac atgaggcagt ggggtatctg tgtgttctcc 180
cactgaccac gctttcttta gtgactcctg attgcctcct caagtcgcag aca 233
<210> 12
<211> 573
<212> DNA
<213> Homo sapiens
<400> 12
ttgacaagga ggatgcagag gaaaaagcaa acttcacctc ttcctaggga aagtgttggc 60
ctgccaacag gaaagaggca acatctggga aaatccccag tctttgccag gaagagtcca 120
tgccaacccc accccatgac ccctgtcctg cctactcatt gtcactcttc actccaatgt 180
ccctccccca gatcctctta taaaatccca ctctttcctg accagacaaa ccataccata 240
tcccaccaga gaggtaagtg ggagctgaga gaagatgaga cccagggagg agctactgca 300
catgacacag gagaatacat gggagggtcc cttcctcagg gagcacagga actctgagac 360
tcagcaaggg tgtcctggga gggctcgggg atgggagagt acacagattc acaactcatt 420
cagaactgta gaagatgatg gatgtgacca agatcacttt agtcctaggg gactagagaa 480
ggaaaatgac atgaggcagt ggggtatctg tgtgttctcc cactgaccac gctttcttta 540
gtgactcctg attgcctcct caagtcgcag aca 573
<210> 13
<211> 1332
<212> DNA
<213> Homo sapiens
<400> 13
ctgcagcctt gaactcctgg gttcaactga aggtcctcct acctcagcct gctgagtagc 60
taggaccaca agcacacacc accgcaactg gcttaaatta aaatataaat tgtagagata 120
gggtcttaat gtgttgccca ggctgctctt gaactccttg cttcaggtga tcctcccacc 180
tcagcctctc aaagtgctgg gattatagac ctgagccaca gcacctggcc aactgaccta 240
tgattttaca caatggctgc tcttcccttc tttaactatt attcattctt ctttgatcct 300
cattatttga ctgtagtcct tcttatgtct tgttttcctt cattacctct tattctatca 360
cattgccatt gtcattctcc actggggaag ctctttcttg ctgaagactg gaaagacaag 420
tccattcacc tgattttctg taagattgtg gctcatgtat tgacttgtca gacaattctg 480
aagtttcatc aaaattagct atcatgcttg cataatggcc ctgaaccctc actcctactc 540
ttagcttcag taccatctat gtcctcaact gtccatgata cttataattc ccgtaaatct 600
tcacttaaca cctaacattt atttaatctt actaggcaag gtaataagaa atacataggt 660
ttgcctccag aagtgggttc ttaagaaacc caccagagga actcctcttt cagatgtcca 720
cattagaaga tttcatatca catttggtgc cacaggcctt tgacaaggag gatgcagagg 780
aaaaagcaaa cttcacctct tcctagggaa agtgttggcc tgccaacagg aaagaggcaa 840
catctgggaa aatccccagt ctttgccagg aagagtccat gccaacccca ccccatgacc 900
cctgtcctgc ctactcattg tcactcttca ctccaatgtc cctcccccag atcctcttat 960
aaaatcccac tctttcctga ccagacaaac cataccatat cccaccagag aggtaagtgg 1020
gagctgagag aagatgagac ccagggagga gctactgcac atgacacagg agaatacatg 1080
ggagggtccc ttcctcaggg agcacaggaa ctctgagact cagcaagggt gtcctgggag 1140
ggctcgggga tgggagagta cacagattca caactcattc agaactgtag aagatgatgg 1200
atgtgaccaa gatcacttta gtcctagggg actagagaag gaaaatgaca tgaggcagtg 1260
gggtatctgt gtgttctccc actgaccacg ctttctttag tgactcctga ttgcctcctc 1320
aagtcgcaga ca 1332
<210> 14
<211> 2056
<212> DNA
<213> Homo sapiens
<400> 14
ctcctatcta gctgtatgtt tgtacgatga actaaactct tttcatctcc cctcacccgt 60
ctcagcctct ggtaactatt ctaccctctg gttaccttct accccgtacc tccatgtgtt 120
tacatattgt tgtataaatt ttgtggaaaa aaatcctttt taacaaaata atctgctggc 180
tatatttctt ctcatattat ttgaggaatt gcattgaatt ttgggtactg aattttgagg 240
acgtgaatta aggatgagct acatctcatg acctcgtcac agacccctac tataaacatc 300

tgcatacaag ttggcatatt acaacaatag catgagagtc agggtctgta gaaattccca 360
gcctaacttt ttcctggact ggccaaactg aacctgaacc agtgtatggg caatgtagta 420
tgcttgtcag aggcaccaaa ctgctaactc aaaaataaac atgactaaaa gaacttctcc 480
cagtataccc atatgaagct gaccatttgc ccagtaagaa tgcaatgaaa agggctttta 540
atgtacagcc aaatggcagt ccagcaagga agctcatgag caacctacaa gtagtgagca 600
ttttgatgta atagagactt acatgcaatt cagaattatg tttacaacat cacaaaacaa 660
aaatttagag ataggctctc actctgttgc ctaagctgga gtgcagtggg atgatcataa 720
cttactgcag ccttgaactc ctgggttcaa ctgaaggtcc tcctacctca gcctgctgag 780
tagctaggac cacaagcaca caccaccgca actggcttaa attaaaatat aaattgtaga 840
gatagggtct taatgtgttg cccaggctgc tcttgaactc cttgcttcag gtgatcctcc 900
cacctcagcc tctcaaagtg ctgggattat agacctgagc cacagcacct ggccaactga 960
cctatgattt tacacaatgg ctgctcttcc cttctttaac tattattcat tcttctttga 1020
tcctcattat ttgactgtag tccttcttat gtcttgtttt ccttcattac ctcttattct 1080
atcacattgc cattgtcatt ctccactggg gaagctcttt cttgctgaag actggaaaga 1140
caagtccatt cacctgattt tctgtaagat tgtggctcat gtattgactt gtcagacaat 1200
tctgaagttt catcaaaatt agctatcatg cttgcataat ggccctgaac cctcactcct 1260
actcttagct tcagtaccat ctatgtcctc aactgtccat gatacttata attcccgtaa 1320
atcttcactt aacacctaac atttatttaa tcttactagg caaggtaata agaaatacat 1380
aggtttgcct ccagaagtgg gttcttaaga aacccaccag aggaactcct ctttcagatg 1440
tccacattag aagatttcat atcacatttg gtgccacagg cctttgacaa ggaggatgca 1500
gaggaaaaag caaacttcac ctcttcctag ggaaagtgtt ggcctgccaa caggaaagag 1560
gcaacatctg ggaaaatccc cagtctttgc caggaagagt ccatgccaac cccaccccat 1620
gacccctgtc ctgcctactc attgtcactc ttcactccaa tgtccctccc ccagatcctc 1680
ttataaaatc ccactctttc ctgaccagac aaaccatacc atatcccacc agagaggtaa 1740
gtgggagctg agagaagatg agacccaggg aggagctact gcacatgaca caggagaata 1800
catgggaggg tcccttcctc agggagcaca ggaactctga gactcagcaa gggtgtcctg 1860
ggagggctcg gggatgggag agtacacaga ttcacaactc attcagaact gtagaagatg 1920
atggatgtga ccaagatcac tttagtccta ggggactaga gaaggaaaat gacatgaggc 1980
agtggggtat ctgtgtgttc tcccactgac cacgctttct ttagtgactc ctgattgcct 2040
cctcaagtcg cagaca 2056
<210> 15
<211> 1057
<212> DNA
<213> Rattus sp.
<400> 15
actagtttat ctaaccctcg tttattaaaa aggatattaa ttttcgtaac tataattttt 60
atatgttggg agtaaaacca ttttgagtgt tttgtccaat gtcacctgac cgacagtttg 120
aatagtcggg ggtagagcct ttcgtatact aaagtccagt ttgtttaacc atattgcttc 180
agtggggttt catgggctca ggaagtaacg aatgaaccag acatagagct atgaaaggta 240
tgtggtgcga gctcagccct tgcgacaaag ctttgagcaa cagcccgcgt gggcttaggg 300
ttgtttgcag ttggtgttag agacctcaca caaagtcatg tggcagataa cccggaggca 360
aaattcaaac ccagtcgcca tatgctcatg tttaacggtg accctgtgca cctttctgat 420
cacatgcttt ggaattgcaa agatctcccc acaaggcaga gtgcagagag aattaaggat 480
gacataaccc tgtgggctgg gctgatctgg gctgctcctc ttggcttagg tgtagaagca 540
tagcagtgaa ttggtgactg atataacgtg tatttattat ctatagtttt gtgtgtgtgt 600
gtgtgtgtgt gtgtgtgtgt gtgtgtgtgt gtgtgtatga tcatatttac acatgattca 660
tctagccttt atgaaaggat gatgaaacca gacatttagc cttgcggtta catgcatact 720
agcaagaaac tcgatatagg atctttttgc tgcgaactct gtacttagga cttagctttg 780
agcaatagcc ccgaaaggtt ttagcactgt ttgcggtcag cacacaaacc gtggttcaaa 840
gctcctcctt attctgaagt ggtctttgtc cttgaacata agatagaagt gacccctgtg 900
ctgttaatta ttggcaaatt gcctaacttc aacataagga aaaaaagtaa catgtttgcc 960
cactgaaggt tactagttaa cagacatcac ttaaaaagga tataaaagaa cttcagcgct 1020
actgctcaca gtatccggct tctaccactg tctggga 1057
<210> 16
<211> 3230
<212> DNA
<213> Rattus sp.
<400> 16
   tcgaggtaaa aggcaagcct tgctattcgg cctacctacc aacttttctt tgggtctctt 60
   ggaaatgtga cttcctctaa aaatacctca ccggtagaaa gacactagga gctgttttcc 120
   ttccacatag caggacatcc atcagagaac ttggatacag tggatgcagt catttttcca 180
   ccagatgaga tgtggtctca gtcagtaatg ctgacactca ttgctgacac ttcccttcag 240
   tgaacaacat ctcatatgcg gacttcacac tttttgttga atgaatcatg gaacccccaa 300
   ctgttgagtt ctacttggtg gcggccctat tctgagtgac cctcttacta gtttatctaa 360
   ccctcgttta ttaaaaagga tattaatttt cgtaactata atttttatat gttgggagta 420
   aaaccatttt gagtgttttg tccaatgtca cctgaccgac agtttgaata gtcgggggta 480
   gagcctttcg tatactaaag tccagtttgt ttaaccatat tgcttcagtg gggtttcatg 540
   ggctcaggaa gtaacgaatg aaccagacat agagctatga aaggtatgtg gtgcgagctc 600
   agcccttgcg acaaagcttt gagcaacagc ccgcgtgggc ttagggttgt ttgcagttgg 660
   tgttagagac ctcacacaaa gtcatgtggc agataacccg gaggcaaaat tcaaacccag 720
   tcgccatatg ctcatgttta acggtgaccc tgtgcacctt tctgatcaca tgctttggaa 780
   ttgcaaagat ctccccacaa ggcagagtgc agagagaatt aaggatgaca taaccctgtg 840
   ggctgggctg atctgggctg ctcctcttgg cttaggtgta gaagcatagc agtgaattgg 900
   tgactgatat aacgtgtatt tattatctat agttttgtgt gtgtgtgtgt gtgtgtgtgt 960
   gtgtgtgtgt gtgtgtgtgt gtatgatcat atttacacat gattcatcta gcctttatga 1020
   aaggatgatg aaaccagaca tttagccttg cggttacatg catactagca agaaactcga 1080
   tataggatct ttaaaggtag gaagatctca gagtggtcaa ggagaggtgt agcacacctg 1140
   taatccagga cccaggagat aggaaaatca ggaactcaaa gccaactgct cacaaaccga 1200
   ccatgcaaac gattgaccaa actaaaatgg agactcttat ttcactttaa acccttgtca 1260
   ctggataaat acattcatta tctactcagc aagtgttggg tcctgtctca acacttgacg 1320
   tgctatgcat agtgtaaaac gtactcagtg tacttagacc atttattgtt attttatcca 1380
   atgagtaggg atgagaggag agggagacag agacagagac agagacagag acagagagag 1440
   acagagacag agagagacag agagagacag agagagacag agagagagac agagaggaga 1500
   gagaggagag agatagagag gacagagaag acagagagaa gagcagtaga cagacacaca 1560
   gagagagaga gagagagaga gagagagaga gagagagaga gagagagaga gagagacaga 1620
   gagagacaga gagagagaca gatagacaca cagagagaga aagagaggga gagagagaca 1680
   cagagagaga ggtagacaga cagacacaca tacacacaga cagacagaca gacagacaca 1740
   cacacacaga gagagagaga gagagagaga gagagagaga gagagagaga gagagagaga 1800
   gagagagaga ggtctgattt cccttgcaat ctagaaagtt aacgttaaac tctggcctgt 1860
   cattgctttg ttctattttg agaacaggaa gaagtgcagg tatggtctga taataaggcc 1920
   ttattgtgtg tgtttcttgg tttctattat taatatgtta tgaaaatctt tccattacat 1980
   caactattaa tctacaaaat cggtttgata gcggcattgc tctccattta atgaatacac 2040
   tatatttatt tctggtgtaa gtcattttgt ttttataatc acatctttaa agtagctact 2100
   cacaggctat gcagatgact cagctgttaa gggccctttc tgctcttcta gaggccctag 2160
   gttcaattcc cagcccacag ggcagctcat aaccacctgt gactccagtt ccgagggatc 2220
   caatgccctc ttctgacctc tgcaggaaac agacatgcac atatgcaacc aaaactgtga 2280
   agcacataaa agaaaagtcg gagttatagt ttagatgcgc actgggaatt ttttttttta 2340
   caagttttcc attatctctg atgtgtagag aacttaaaga aactgtcata gactctggga 2400
   caccgagagt gaaggtatga gatgaagccc tcctagggtc agagtatatc agtgctcgtt 2460
   agttagtgtt agcatcatca tcccgagggc ctgagaggat gctgcagcta acccatgcat 2520
   ctgtgacata catgcagaat tagcctttgg tacacgacat tagaatggga actcgttcat 2580
   caggctttgt gctgtaagtt ttctatgtta aaccagacac gaaacacaaa attaaataca 2640
   cccccttgac caatggtttt gagctaaata acaactggat aattaagaaa tgcacccact 2700
   gatgaagagt ctgaatgact cagacatacg ttcagcgcct agcaggcaac atgcatttta 2760
   ctattttttt taaaaagaat taattcattt agagctacat agtaagcttg catattttct 2820
   ttatggctgt atcaatgttt tacattgaaa taaataaatc tttcttcagc ttaggagata 2880
   ctttctattg gaagagttta gttggcggag aacatttgca gcatttgcaa ggttttgttt 2940
   tttttccact ctgaagtggt ctttgtcctt gaacataaga tagaagtgac ccctgtgctg 3000
   ttaattattg gcaaattgcc taacttcaac ataaggaaaa aaagtaacat gtttgcccac 3060
   tgaaggttac tagttaacag acatcactta aaaaggatat aaaagaactt cagcgctact 3120
   gctcacagta tccggcttct accactgtct gggatgaagc agccagcaac catgaagtgg 3180
   agcgcatcca tttccttcct tctcctgcta aattttgctg aacccagagt 3230
<210> 17
<211> 30
<212> DNA
<213> artificial sequence
<220>
<223> primer
<400> 17
cggggtacca ctgcagcctt gaactcctgg 30
<210> 18
<211> 30
<212> DNA
<213> artificial sequence
<220>
<223> primer
<400> 18
acggtcgact gtctgcgact tgaggaggca 30
<210> 19
<211> 31

<212> DNA
<213> artificial sequence
<220>
<223> primer
<400> 19
ccgcgtcgag ctgcagattt tccagttagt c 31
<210> 20
<211> 30
<212> DNA
<213> artificial sequence
<220>
<223> primer
<400> 20
tcgacgcgtg tgaggacaga gatggctgtg 30